# EUROPEAN PATENT SPECIFICATION

(11) **EP 0 910 563 B1**
(45) Date of publication and mention of the grant of the patent: **02.05.2003**
(21) Application number: 96923553.0
(22) Date of filing: 28.06.1996
(51) Int. Cl.: C07D 235/04, C07D 235/06, C07C 235/08, C07D 211/06, C07D 211/08, C07D 213/02

(54) **INTEGRIN RECEPTOR ANTAGONISTS**
INTEGRIN-REZEPTOR-ANTAGONISTEN
ANTAGONISTES DU RECEPTEUR DE L'INTEGRINE

(30) Priority: 29.06.1995 US 665; 25.08.1995 US 2788
(43) Date of publication of application: 28.04.1999
(73) Proprietor: SMITHKLINE BEECHAM CORPORATION, Philadelphia, Pennsylvania 19103 (US)
(72) Inventor: BONDINELL, William, E., Wayne, PA 19087 (US); MILLER, William, H., Schwenksville, PA 19473 (US)
(74) Representative: Giddings, Peter John, Dr.
(86) International application number: US9611108
(87) International publication number: WO97001540

(56) References cited:
- US-A- 3 906 040
- US-A- 3 972 936
- US-A- 5 302 602

## Description

This invention relates to pharmaceutically active compounds which bind to integrins, such as the vitronectin receptor and fibrinogen receptor. Such compounds are useful for inhibiting platelet aggregation and osteoclast attachment to bone.

### BACKGROUND OF THE INVENTION

Integrins are a family of heterodimeric proteins which generally mediate cell adhesion. Typical of such proteins are the vitronectin receptor (an α_{V}β₃ heterodimer) and the fibrinogen receptor (an α_{IIb}β₃ heterodimer). The natural ligands of these receptors (e.g., vitronectin and fibrinogen) have been found to share a common -Arg-Gly-Asp- amino acid sequence, which appears to be critical for binding. In fact, many of the integrin receptors appear to cross react with ligands which possess such an amino acid sequence. For instance, the α_{IIb}β₃ receptor reacts with fibronectin and vitronectin, thrombospondin and von Willebrand factor, as well as fibrinogen. Functionally fibrinogen, a dimer having two binding sites for α_{IIb}β₃, reacts with activated receptors found on the surface of platelets. The binding of α_{IIb}β₃ receptors on adjacent platelets, by fibrinogen leads to crosslinking and is considered to be a major factor in platelet aggregation. Compounds which inhibit the binding of the α_{IIb}β₃ receptor to fibrinogen have been shown to inhibit the platelet aggregation *in vitro,* and thrombus formation in vivo. See, for instance, EP-A 0 341 915.

The vitronectin receptor is found on a variety of cell types, such as on osteoclasts and the endothelial cells lining blood vessels. Recent studies have indicated that the attachment of osteoclasts to the bone matrix is mediated through these cell surface adhesion receptors. For instance, Davies, *et al., J. Cell Biol.*, **1989,** *109*, 1817, disclose that the osteoclast functional antigen, which is implicated in the regulation of bone resorption, is biochemically related to the vitronectin receptor. The vitronectin receptor is known to bind to bone matrix proteins, such as osteopontin, bone sialoprotein and thrombospondin, which contain the tri-peptide Arg-Gly-Asp (or RGD) motif. Thus, Horton, *et al., Exp. Cell Res.* **1991**, *195*, 368, disclose that RGD-containing peptides and an anti-vitronectin receptor antibody (23C6) inhibit dentine resorption and cell spreading by osteoclasts. Bertolini *et al., J. Bone Min. Res*., 6, Sup. 1, S146, 252 have shown that cyclo-S,S-N^{α}-acetyl-cysteinyl-N^{α}-methyl-argininyl-glycyl-aspartyl-penicillamine amide inhibits osteoclast attachment to bone. In addition, Sato, *et al., J. Cell Biol.* **1990**, *111*, 1713 disclose that echistatin, a snake venom peptide which contains the RGD sequence, is a potent inhibitor of bone resorption in tissue culture, and inhibits attachment of osteoclasts to bone. Fisher, *et al., Endocrinology* **1993,** *132*, 1411, has further shown that echistatin inhibits bone resorption *in vivo* in the rat. EP 528 587 and 528 586 report substituted phenyl derivatives which inhibit osteoclast mediated bone resorption.

Bondinell, *et al.*, in WO 93/00095 (PCT/US92/05463) and WO 94/14776 (PCT/US93/12436) disclose that certain compounds which have a substituted 6-7 bicyclic ring system are useful for inhibiting the fibrinogen (α_{IIb}β₃) receptor. Other 6-7 bicyclic ring systems which inhibit the fibrinogen receptor are disclosed by Blackburn *et al.* in WO 93/08174 (PCT/US92/08788). Blackburn *et al.*, WO 95/04057 (PCT/US94/07989) also disclose compounds which have a five- or six-membered ring fused to such 6-7 bicyclic ring to form a tricyclic ring system, which are useful as antagonists of the fibrinogen receptor. Other compounds having 6-7 bicyclic ring systems that selectively inhibit the vitronectin receptor are disclosed in WO 96/00730 (PCT/US95/08306) and WO 96/00574 (PCT/US95/08146). US 3,906,040 (Vincent et al) describes a series of substituted dibenzo (a,d) cycloheptenes which are claimed to have uses in the cardiovascular field. US 5,302,602 (Oshima et al) describes tricyclic compounds having the potential of antagonising thromboxane A2 which are claimed to have antiallergic and/or antihistamic activity. US 3,972,936 (Christy) describes tetrafluoro derivatives of 10,11-dihydro-5H-dibenzo[a,d]cycloheptene which are claimed to have antidepressant activity. It has now been discovered that certain new tricyclic ring systems are useful templates for preparing integrin receptor antagonists. It has also been discovered that such a ring system may be used as a template, which may be suitably substituted to prepare compounds which are selective for either the fibrinogen receptor or the vitronectin receptor.

### SUMMARY OF THE INVENTION

It is an object of this invention to provide compounds of the formula (IV), as described hereinafter, which have pharmacological activity for the inhibition of integrin receptors. It is an object of this invention to provide a template which may be suitably substituted to provide selective binding for specific integrin receptors, especially the fibrinogen (α_{IIb}β₃) or the vitronectin (α_{V}β₃) receptor relative to each other and other integrin receptors.

This invention is also a pharmaceutical composition comprising a compound according to formula (IV) and a pharmaceutically carrier.

This invention is also a use of compounds of formula (IV) in the manufacture of a medicament for the treatment of diseases in which the pathology may be modified by binding to an integrin receptor, especially the vitronectin or the fibrinogen receptor. In a particular aspect, the compounds of this invention are useful in the manufacture of a medicament for treating osteoporosis, atherosclerosis, restenosis, cancer and conditions in which it is desirable to inhibit platelet aggregation, such as stroke, transient ischemia attacks, myocardial infarction and rethrombosis following thrombolytic therapy.

### DETAILED DESCRIPTION

This invention comprises compounds of formula (IV): 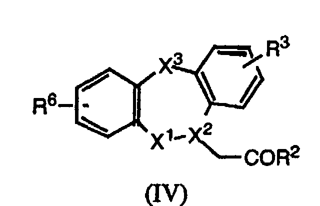 wherein
X¹-X² is CHR¹-CH, CR¹=CH, NR¹-CH, S(O)ᵤ-CH or O-CH;
X³ is CR⁵R⁵', NR⁵, S(O)ᵤ or O;
R' is H, C₁₋₆alkyl, C₃₋₇cycloalkyl-C₀₋₄alkyl or Ar-C₀₋₄alkyl;
R" is R', -C(O)R' or -C(O)OR⁵;
R"' is C₁₋₆alkyl, C₃₋₇cycloalkyl-C₀₋₄alkyl or Ar-C₀₋₄alkyl;
R¹ is H, C₁₋₆alkyl, C₃₋₇cycloalkyl-C₀₋₄alkyl or Ar-C₀₋₄alkyl;
R² is -OR', -NR'R", -NR'SO₂R"', -NR'OR', -OCR'₂C(O)OR', -OCR'₂OC(O)-R', -OCR'₂C(O)NR'₂, CF₃ or -COCR'₂R^{2'};
R^{2'} is -OR', -CN, -S(O)ᵣR', S(O)₂NR'₂, -C(O)R'C(O)NR'₂ or -CO₂R';
R⁵ and R^{5'} are independently H, C₁₋₆alkyl, C₃₋₇cycloalkyl-C_{O-4}alkyl or Ar-C₀₋₄alkyl;
R⁶ is W'-(CR'₂)_{q}-U selected from: 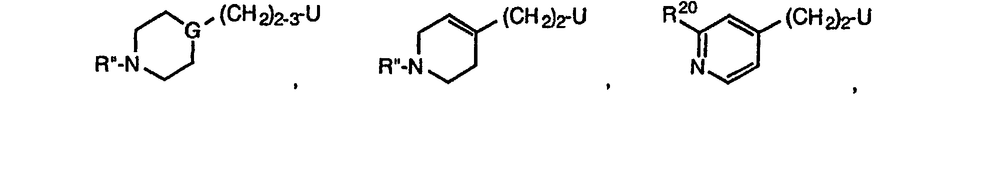 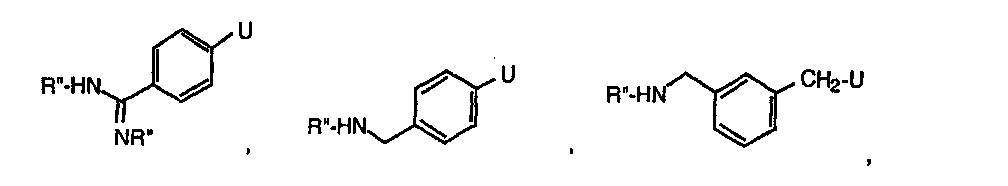 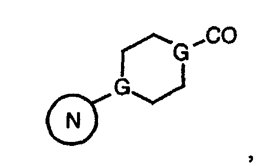
R"HNC(=NH)NH-(CH₂)₃(CHR¹⁰)-U and R"HN-(CH₂)₅-U wherein G is N or CH, R²⁰ is hydrogen, amino, mono or di-C₁₋₄alkylamino, hydroxy or C₁₋₄alkyl, and U is NRCO, CONR', (CH₂)CO, CH=CH, C≡C, CH₂O, OCH₂ or (CH₂)₂; or R⁶ is W'-(CR'₂)_{q}-U wherein W' is 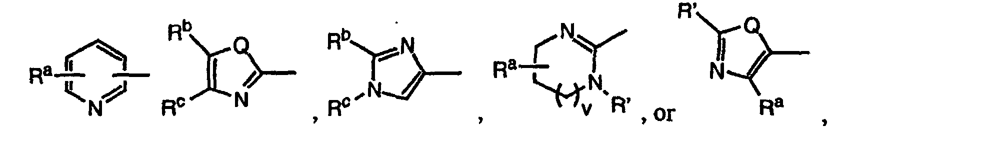 where Q is NH; R^{a} is C₁₋₆alkyl, C₁₋₆alkoxy, halogen or R'NH, R^{b} and R^{c} are joined to form a cyclohexyl, phenyl or pyridyl ring optionally substituted by halogen, C₁₋₄alkyl, OR¹, SR¹, COR¹, OH, NO₂, N(R¹)₂, CO(NR¹)₂, CH₂N(R¹)₂, CN, or R"R'NC(=NR')- and -(CR'₂)_{q}-U- is (CH₂)_{q}-NR'CO, (CH₂)_{q}-CH₂O or (CH₂)_{q}-CH₂CH₂;
R³ is H, halo, -OR¹², -SR¹², -CN, -NR'R¹², -NO₂, -CF₃, CF₃S(O)ᵣ-, -CO₂R', -CONR'₂, R¹⁴-C₀₋₆alkyl-, R¹⁴-C₁₋₆oxoalkyl-, R¹⁴-C₂₋₆alkenyl-, R¹⁴-C₂₋₆alkynyl-, R¹⁴-C₀₋₆alkyloxy-, R¹⁴-C₀₋₆alkylamino- or R¹⁴-C₀₋₆alkyl-S(O)ᵣ-;
R¹⁰ is H, C₁₋₄alkyl or -NR'R";
R¹² is R', -C(O)R', -C(O)NR'₂, -C(O)OR⁵, -S(O)ₘR' or S(O)₂NR'₂;
R¹⁴ is H, C₃₋₆cycloalkyl, Het or Ar;
m is 1 or 2;
q is 0, 1, 2 or 3;
r is 0, 1 or 2;
u is 0, 1 or 2; and
v is 0 or 1;
Ar represents phenyl or naphthyl optionally substituted by one to three of C₁₋₄alkyl, C₁₋₄alkoxy, C₁₋₄alkthio, trifluoroalkyl, OH, F, Cl, Br or I.

Het represents a five or six membered monocyclic ring, or a nine or ten-membered bicyclic ring containing one to three heteroatoms chosen from the group of nitrogen, oxygen and sulfur optionally substituted by one to three of C₁₋₄alkyl, C₁₋₄alkoxy, C₁₋₄alkthio, trifluoroalkyl, OH, F, Cl, Br or I.

Ⓝ represents a nitrogen heterocycle, which may be a saturated or unsaturated stable five-, six- or seven-membered monocyclic ring, or a seven- to ten-membered bicyclic ring containing up to three nitrogen atoms or containing one nitrogen atom and a heteroatom chosen from oxygen and sulfur, and which may be substituted by H, C₁₋₄alkoxy, F, Cl, Br, I, NO₂, NR'₂, OH, CO₂R', CONHR', CF₃, R¹⁴-C₀₋₄alkyl, R¹⁴-C₁₋₄alkyl-S(O)ᵤ or C₁₋₄alkyl substituted by any of the aforementioned substituents;
C₃₋₇cycloalkyl refers to a carbocyclic system of three to seven carbon atoms, which may contain up to two unsaturated carbon-carbon bonds optionally substituted by up to three substituents, such as chosen from R³, on the cycloalkyl ring; or a pharmaceutically acceptable salt thereof.

In cases wherein the compounds of this invention may have one or more chiral centers, unless specified, this invention includes each unique nonracemic compound which may be synthesized and resolved by conventional techniques. In cases in which compounds have unsaturated carbon-carbon double bonds, both the cis (Z) and trans (E) isomers are within the scope of this invention. In cases wherein compounds may exist in tautomeric forms, such as keto-enol tautomers, such as 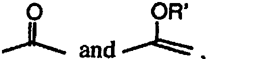 and tautomers of guanidine-type groups, such as 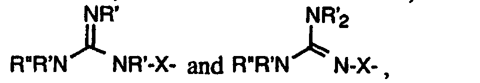 each tautomeric form is contemplated as being included within this invention whether existing in equilibrium or locked in one form by appropriate substitution with R'. The meaning of any substituent at any one occurrence is independent of its meaning, or any other substituent's meaning, at any other occurrence, unless specified otherwise. In particular, the compound may be of the formulae (V-1) to (V-9):

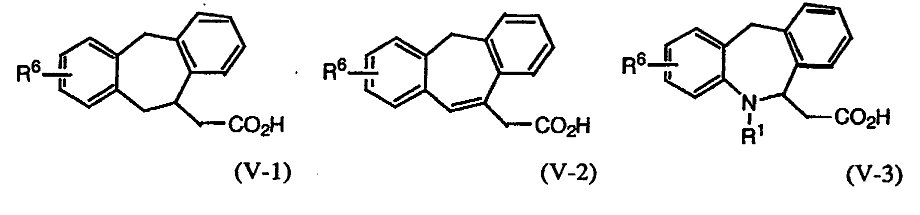

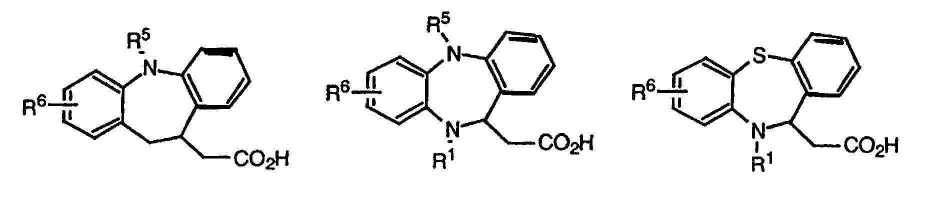

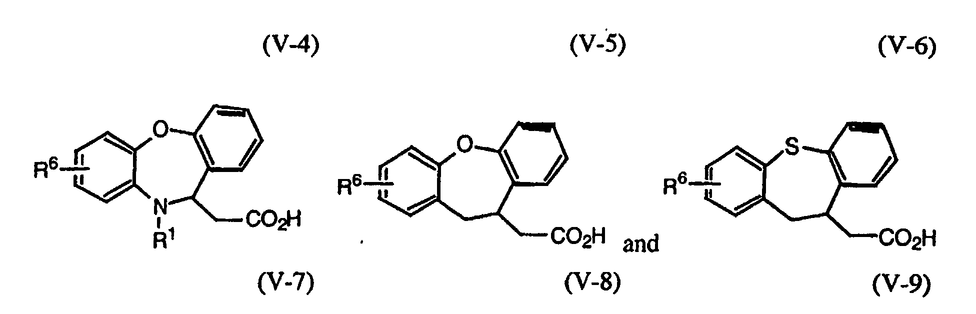

Preferably X¹-X² is CHR¹-CH or NR¹-CH.

Suitably, X³ is CR⁵R^{5'}. Preferably, X³ is CH₂.

Suitably R¹ is H. Suitably, R² is OR'. Suitably, R³ is H.

Particularly good substituents for promoting selective fibrinogen antagonist activity are: 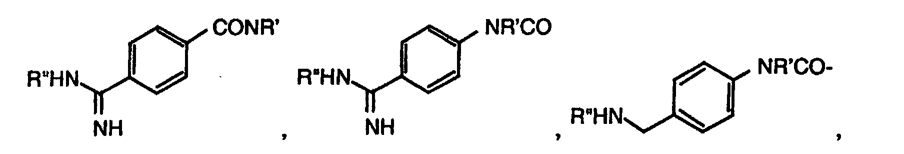 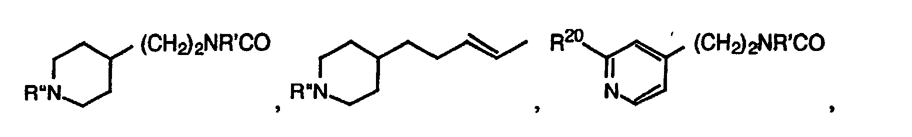 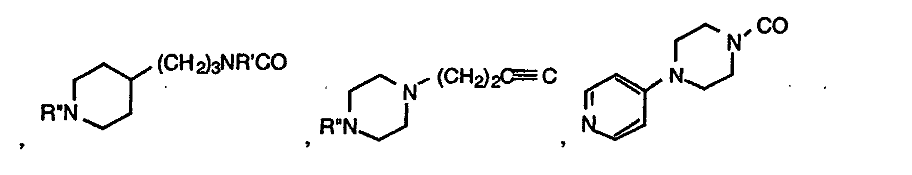 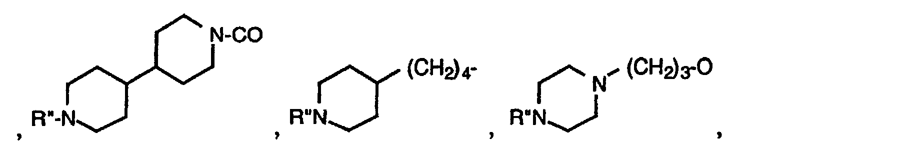 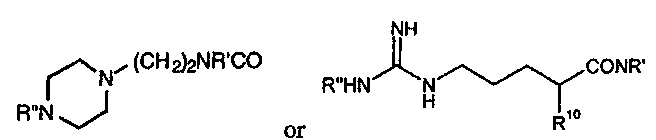 wherein R' are H or C₁₋₄alkyl. Preferably R' is methyl and R" is H.

Particularly preferred of such groups for R⁶ are:

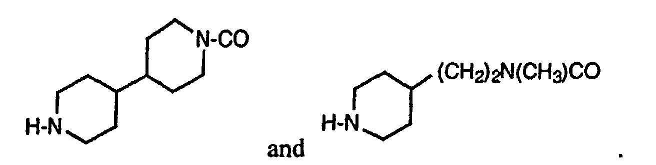

Specific preferred R⁶ substituents for enhancing vitronectin activity are

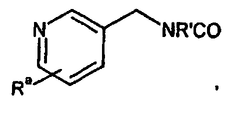

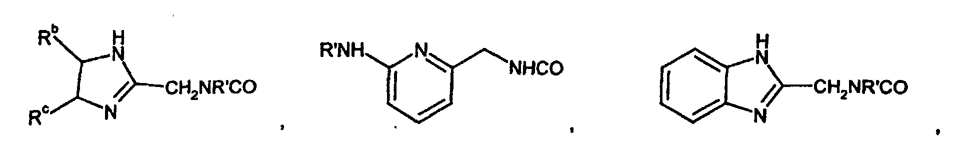

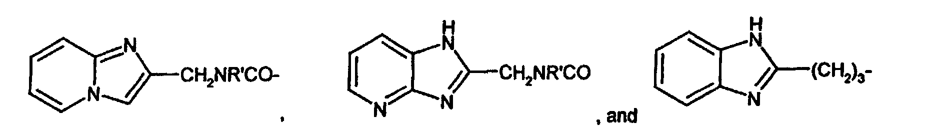

By appropriate selection of the spacing of the substituent W and/or W' from the phenyl ring of the 6-7 ring system, compounds having selective activity for either the vitronectin and fibrinogen receptor, or dual activity for both receptors, may be obtained.. In general, fibrinogen antagonist activity will be favored by an intramolecular distance of about 16 angstroms between the oxygen of the carbonyl moiety attached to the seven-membered ring, and the basic nitrogen moiety of W or W'; while vitronectin antagonist activity will be favored by about 14 angstroms between the respective acidic and basic centers.

Specific compounds of this invention are:
(±)-10,11-Dihydro-3-[[[(1H-benzimidazole-2-yl)methyl]amino]carbonyl]-5H-dibenzo[a,d]cycloheptene-10-acetic acid;
(±)-10,11-Dihydro-3-[[[(4-aza-5-methyl-1H-benzimidazole-2-yl)methyl]methylamino] carbonyl]-5H-dibenzo[a,d]cycloheptene-10-acetic acid;
(±)-10,11-Dihydro-3-[[[(1H-benzimidazole-2-yl)methyl]methylamino]carbonyl]-5H-dibenzo[a,d]cycloheptene-10-acetic acid;
(±)-10,11-Dihydro-3-[1-(4,4'-bipiperidinyl)carbonyl]-5H-dibenzo[a,d]cycloheptene-10-acetic acid;
(±)-10,11-Dihydro-3-[3-(2-benzimidazolyl)-1-propyl]-5H-dibenzo[a,d]cycloheptene-10-acetic acid;
(±)-10,11-Dihydro-3-[[[2-(2-pyridylamino)ethyl]amino]carbonyl]-5H-dibenzo[a,d]cycloheptene-10-acetic acid;
(±)-10,11-Dihydro-3-[3-(2-pyridylamino)-1-propyloxy]-5H-dibenzo[a,d]cycloheptene-10-acetic acid; and
2-[[[(1H-Benzimidazol-2-yl)methyl]methylamino]carbonyl]-6,11-dihydro-5H-dibenz[b,e]azepine-6-acetic Acid.

Abbreviations and symbols commonly used in the peptide and chemical arts are used herein to describe the compounds of this invention.

C₁₋₄alkyl as applied herein is meant to include methyl, ethyl, n-propyl, isopropyl, n-butyl, isobutyl and t-butyl. C₁₋₆alkyl additionally includes pentyl, n-pentyl, isopentyl, neopentyl and hexyl and the simple aliphatic isomers thereof. Any C₁₋₄alkyl or C₁₋₆alkyl group may be optionally substituted by R⁷ unless otherwise indicated. C₀₋₄alkyl and C₀₋₆alkyl additionally indicates that no alkyl group need be present (*e.g*., that a covalent bond is present).

C₂₋₆ alkenyl as applied herein means an alkyl group of 2 to 6 carbons wherein a carbon-carbon single bond is replaced by a carbon-carbon double bond. C₂₋₆alkenyl includes ethylene, 1-propene, 2-propene, 1-butene, 2-butene, isobutene and the several isomeric pentenes and hexenes. Both cis and trans isomers are included. Any C₂₋₆alkenyl group may be optionally substituted by R⁷ unless otherwise indicated.

C₂₋₆ alkynyl means an alkyl group of 2 to 6 carbons wherein one carbon-carbon single bond is replaced by a carbon-carbon triple bond. C₂₋₆ alkynyl includes acetylene, 1-propyne, 2-propyne, 1-butyne, 2-butyne, 3-butyne and the simple isomers of pentyne and hexyne. Any sp³ carbon atom in the C₂₋₆alkynyl group may be optionally substituted by R⁷.

C₁₋₄oxoalkyl refers to an alkyl group of up to four carbons wherein a CH₂ group is replaced by a C(O), or carbonyl, group. Substituted formyl, acetyl, 1-propanal, 2-propanone, 3-propanal, 2-butanone, 3-butanone, 1- and 4-butanal groups are representative. C₁₋₆oxoalkyl includes additionally the higher analogues and isomers of five and six carbons substituted by a carbonyl group. C₃₋₆oxoalkenyl and C₃₋₆oxoalkynyl refers to a C₃₋₆alkenyl or C₃₋₆alkynyl group wherein a CH₂ group is replaced by C(O) group. C₃₋₄oxoalkenyl includes 1-oxo-2-propenyl, 3-oxo-1-propenyl, 2-oxo-3-butenyl and the like.

A substituent on a C₁₋₆ alkyl, C₂₋₆ alkenyl, C₂₋₆ alkynyl or C₁₋₆ oxoalkyl group, such as R⁷, may be on any carbon atom which results in a stable structure, and is available by conventional synthetic techniques.

R¹⁴-C₁₋₆ alkyl refers to a C₁₋₆ alkyl group wherein in any position a carbon-hydrogen bond is replaced by a carbon-R¹⁴ bond. R¹⁴-C₂₋₆ alkenyl and R¹⁴-C₂₋₆ alkynyl have a similar meaning with respect to C₂₋₆ alkenyl and C₂₋₆ alkynyl.

Illustrative heterocycles for Het are benzofuran, benzimidazole, benzopyran, benzothiophene, furan, imidazole, indole, indoline, morpholine, piperidine, piperazine, pyrrole, pyrrolidine, tetrahydropyridine, pyridine, thiazole, thiophene, quinoline, isoquinoline, and tetra- and perhydro- quinoline and isoquinoline. A six membered ring heterocycle containing one or two nitrogens, such as piperidine, piperazine, tetrahydropyridine and pyridine, are preferred heterocycles for the moiety Z.

Typical of C₃₋₇cycloalkyl are cyclopropyl, cyclobutyl, cyclopentyl, cyclopentenyl, cyclohexyl, cyclohexenyl and cycloheptyl.

Representative of Ⓝ are pyrroline, pyrrolidine, imidazole, imidazoline, imidazolidine, pyrazole, pyrazoline, pyrazolidine, piperidine, piperazine, morpholine, pyridine, pyridinium, tetrahydropyridine, tetrahydro- and hexahydro-azepine, quinuclidine, quinuclidinium, quinoline, isoquinoline, and tetra- and perhydro- quinoline and isoquinoline. In particular, Ⓝ may be pyridyl, pyrolidinyl, piperidinyl, piperazinyl, azetidinyl, quinuclidinyl or tetrahydropyridinyl. Ⓝ is preferably 4-pyridyl, 4-(2-amino-pyridyl), 4-tetrahydropyridyl, 4-piperidinyl or 4-piperazinyl. Benzimidazolyl, 4-azabenzimidazolyl, 5-azabenzimidazolyl and substituted derivatives thereof are preferred moieties for W'.

Certain radical groups are abbreviated herein. t-Bu refers to the tertiary butyl radical, Boc refers to the t-butyloxycarbonyl radical, Fmoc refers to the fluorenylmethoxycarbonyl radical, Ph refers to the phenyl radical, Cbz refers to the benzyloxycarbonyl radical, BrZ refers to the o-bromobenzyloxycarbonyl radical, C1Z refers to the o-chlorobenzyloxycarbonyl radical, Bn refers to the benzyl radical, 4-MBzl refers to the 4-methyl benzyl radical, Me refers to methyl, Et refers to ethyl, Ac refers to acetyl, Alk refers to C₁₋₄alkyl, Nph refers to 1- or 2-naphthyl and cHex refers to cyclohexyl. MeArg is N^{α}-methyl arginine. Tet refers to 5-tetrazolyl.

Certain reagents are abbreviated herein. DCC refers to dicyclohexylcarbodiimide, DMAP refers to dimethylaminopyridine, DIEA refers to diisopropylethylamine, EDC refers to N-ethyl-N'(dimethylaminopropyl)-carbodiimide. HOBt refers to 1-hydroxybenzotriazole, THF refers to tetrahydrofuran, DMF refers to dimethyl formamide, NBS refers to N-bromo-succinimide, Pd/C refers to a palladium on carbon catalyst, DPPA refers to diphenylphosphoryl azide, BOP refers to benzotriazol-1-yloxy-tris(dimethylamino)phosphonium hexafluorophosphate, HF refers to hydrofluoric acid, PPA refers to polyphosphoric acid, TEA refers to triethylamine, TEA refers to trifluoroacetic acid, PCC refers to pyridinium chlorochromate.

A particularly useful intermediate in the preparation of compounds of formula (IV) is a compound of formula (X): 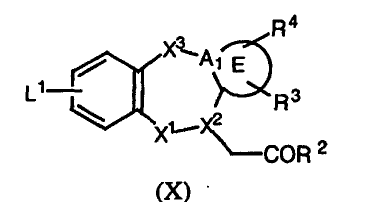 wherein X¹, X², X³, R² and R³ are as defined in formula (IV), R⁴ represents hydrogen, A₁ represents C, E represents phenyl and L¹ is meta to the ring junction and is CHO, CO₂R', Br, I, OH, CF₃SO₃, CH₂-T or NR'R¹⁵, and T is OH, NHR¹⁵, Cl, Br or I, where R¹⁵ is H, C₁₋₁₀ alkyl, C₃₋₇ cycloalkyl-C₀₋₈ alkyl or Ar-C₀₋₈ alkyl. Preferably, L¹ is OH, CF₃SO₃, CO₂R' or NR'R" and R¹ is H, C₁₋₄alkyl, C₁₋₄oxoalkyl, R² is C₁₋₆alkyl, or benzyl, and R⁵/R^{5'} are H,H. Preferably, X¹-X² is CHR¹-CH or NR¹-CH, and X³ is CHR⁵.

The compounds of formula (IV) are generally prepared by coupling the intermediate of formula (X) with a compound of formula (XI):

R^{6'}-L² (XI)

wherein R^{6'} is W'-(CR'₂)_{q}-L², where W', R', and q are as defined in formula (IV), and L² is OH, NHR¹⁵, C≡C , CHO, CO₂R', Br, I or Cl and R¹⁵ is H, C₁₋₁₀ alkyl, C₃₋₇ cycloalkyl-C₀₋₈ alkyl or Ar-C₀₋₈ alkyl. In certain cases, it may be desirable to further modify the group W or W' by appropriate reactions to introduce a functional group, or remove a protecting group, as further illustrated herein. The coupling will generally result in the formation of the U group, and methods for such coupling reactions are well known in the art. WO 93/08174 (PCT/US92/08788; Genentech), WO 93/08174 (PCT/US92/08788; Genentech), WO 96/00730 (PCT/US95/08306; SmithKline Beecham), WO 96/00574 (PCT/US95/08146; SmithKline Beecham), WO 93/00095 (PCT/US92/05463; SmithKline Beecham) and WO 94/14776 (PCT/US93/12436; SmithKline Beecham) generally disclose such reactions and are incorporated herein by reference.

Compounds of formula X may be prepared by the method described in Scheme 1: 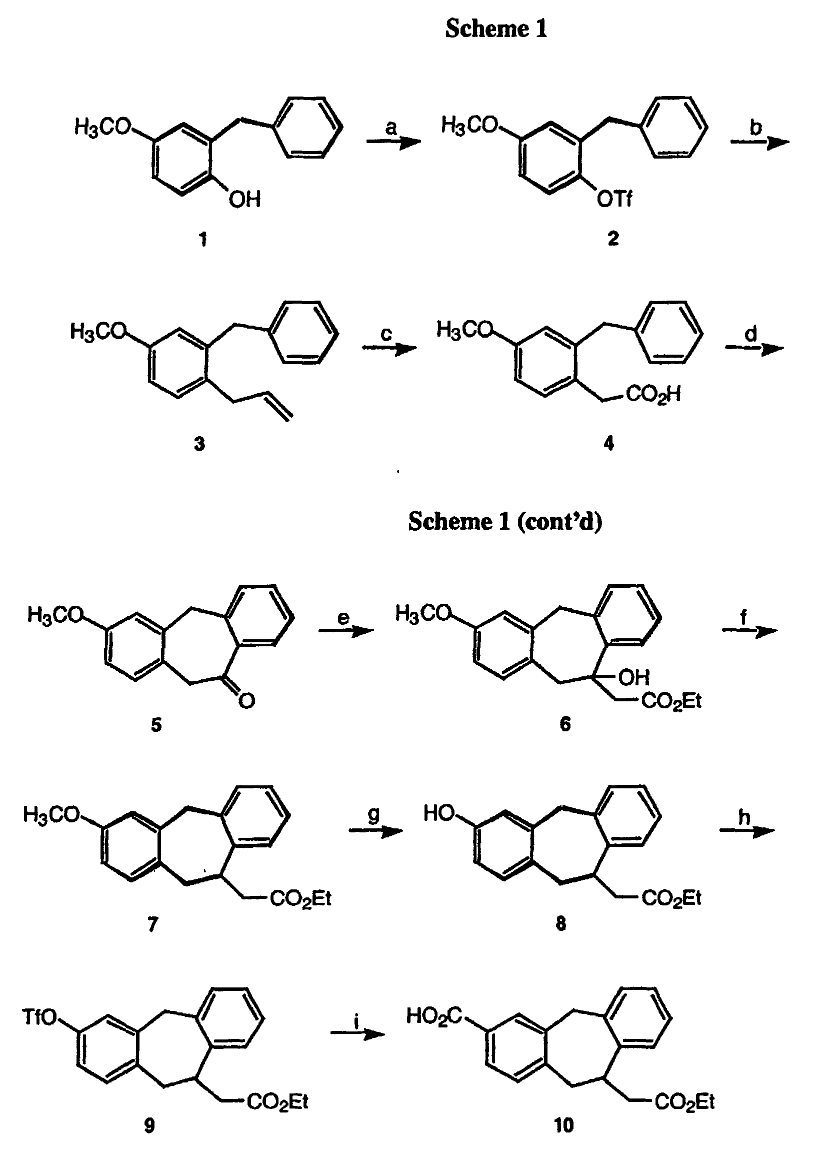 a) Tf₂O, 2,6-lutidine, CH₂Cl₂; b) allyltributyltin, LiCl, (Ph₃P)₂PdCl₂, DMF; c) RuCl₃, H₅IO₆, CCl₄, CH₃CN, H₂O; d) PPA; e) EtOAc/LiHMDS, THE; f) H₂, 10% Pd/C, conc. HCI, AcOH; g) EtSH, AlCl₃, CH₂Cl₂; h) Tf₂O, 2,6-lutidine, CH₂Cl₂; i) CO, Pd(OAc)₂, KOAc, dppf, DMSO.

2-Benzyl-4-methoxyphenol (*J. Am. Chem. Soc.* 1949, *71*, 64) is converted to the corresponding trifluoromethanesulfonate ester, compound 2-Scheme 1(e.g., **1-2)** by reaction with trifluoromethanesulfonic anhydride (Tf₂O) in the presence of a suitable base, for instance 2,6-lutidine, in an inert solvent, generally CH₂Cl₂. Reaction of **1-2** with allyltributyltin in the presence of LiCl and a palladium catalyst, for example bis(triphenylphosphine)palladium (II) chloride ((Ph₃P)₂PdCl₂), in an inert solvent such as DMF, by the method described by Tilley (*J. Org. Chem.* 1990, *55*, 906), affords **1-3**. Oxidative cleavage of the olefin in 3-Scheme 1 to afford directly the carboxylic acid **1-4** can be accomplished by reaction with an appropriate oxidizing agent, classically KMnO₄, in a suitable aqueous solvent, such as aqueous acetone or aqueous acetic acid. Preferably, however, oxidative cleavage of the olefin in **1-3** to afford directly the carboxylic acid **1-4** is conducted according to the general method of Sharpless (*J. Org. Chem.* 1981, *46*, 3936; *J. Org. Chem*. 1985, *50*, 1560, footnote 4), wherein RuO₄ is generated in situ by the reaction of RuCl₃ or RuO₂ with NaIO₄ or H₅IO₆ in a solvent mixture of CCl₄, CH₃CN, and H₂O. Alternatively, the oxidation might be conducted in two operations, involving in the first stage an oxidative cleavage of the olefin to the corresponding aldehyde, which can be accomplished by procedures well known to those of skill in the art, followed by oxidation of the aldehyde to the carboxylic acid using, for example, NaClO₂ as described by Pinnick (*Tetrahedron* 1981, *37*, 2091) or by Dalcanale and Montanari (*J. Org. Chem.* 1986, *51*, 567). Cyclization of **1-4** to **1-5** can be accomplished using polyphosphoric acid, according to the method described by Proctor, Renfrew, and Savage (*J. Chem. Soc.* (C) 1969, 1000). Alternatively, **1-4** can be converted to **1-5** via the corresponding acid chloride of **1-4,** which can be prepared by methods well-known to those of skill in the art. Treatment of this acid chloride with an appropriate Friedel-Crafts catalyst, such as AlCl₃ or SnCl₄, in an inert solvent, such as CH₂Cl₂ or CS₂, provides the cyclic ketone **1-5.** Reaction of **1-5** in an aldol-type reaction with the enolate of ethyl acetate, which can be generated from ethyl acetate on exposure to an appropriate amide base, for instance lithium diisopropylamide (LDA) or lithium bis(trimethylsilyl)amide (LiHMDS), gives **1-6.** Frequently, THF is the solvent of choice for an aldol reaction, although THF in the presence of various additives, for instance HMPA or TMEDA, is often used. Reduction of **1-6** to give **1-7** can be accomplished by hydrogenolysis over an appropriate catalyst, for example palladium metal on activated carbon (Pd/C), in an appropriate solvent, such as acetic acid, in the presence of a mineral acid such as HCl. Alternatively, this reduction can be accomplished by treatment of **1-6** with triethylsilane in the presence of boron trifluoride etherate by the general method of Orphanopoulos and Smonu *(Synth. Commun.* 1988, 833). Removal of the methyl ether of **1-7** to give **1-8** can be accomplished with BBr₃ in an inert solvent, for example CH₂Cl₂, of by reaction with ethanethiol and AlCl₃ in an inert solvent, preferably CH₂Cl₂. Other useful methods for removal of a methyl ether are described in Greene, "Protective Groups in Organic Synthesis" (published by John Wiley and Sons). **1-9**, the trifluoromethanesulfonate ester of **1-8**, prepared by the method described earlier for the conversion of **1-1** to **1-2**, reacts with carbon monoxide in the presence of potassium acetate, 1,1'-bis(diphenylphosphino)ferrocene (dppf), and a palladium catalyst, for instance palladium acetate (Pd(OAc)₂), in a suitable solvent, preferably DMSO, according to the general method described by Cacchi and Lupi (*Tet. Lett*. 1992, *33*, 3939), to give **1-10**.

It will be apparent from the above description that if one dehydrates compound **1-6** instead of performing a hydrogenation, one obtains compounds of the general formula (V-2).

Compounds of formula (IV) wherein X³ is NR⁵, O or S(O)₀₋₂ are prepared using the general method of Scheme 1, except, for example, by substituting compound **1-4** with a 4-methoxy-2-(phenylamino)-, 2-(phenyloxy)-, or 2-(phenylthio)-phenylacetic acid that may be prepared by methods known to the art.

Compounds of formula (X) wherein X¹-X² is NR¹-CH and X³ is CR⁵CR^{5'}, NR', O or S(O)₀₋₂ are prepared using the general method described in Scheme 2: 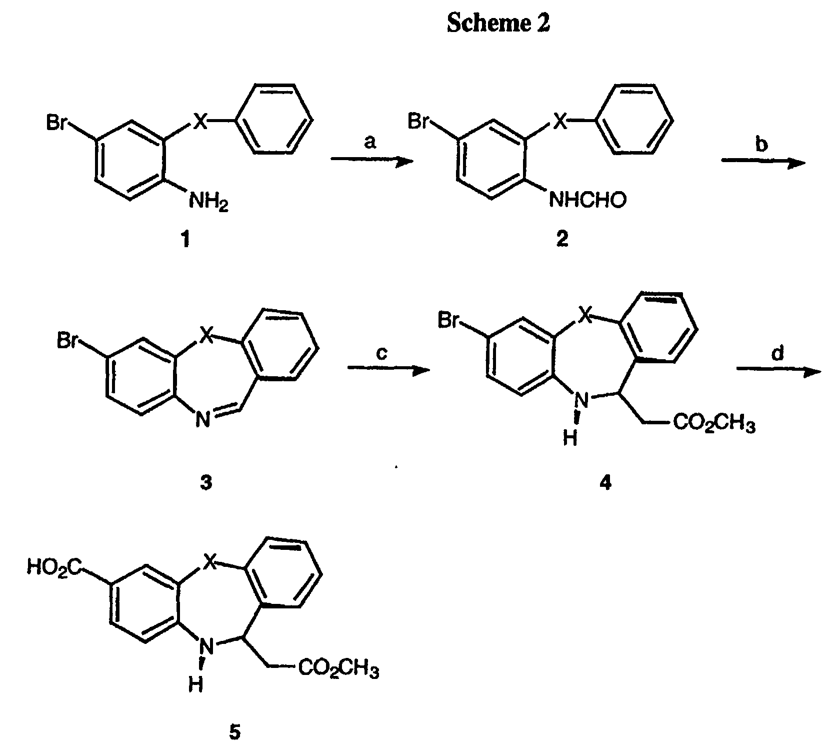 a) HCO₂H, AC₂O, Δ; b) POCl₃, PPA, Δ; c) CH₂=C(OCH₃)Si(CH₃)₂-t-Bu, TMSCN, [Rh(COD)Cl]₂, CH₂Cl₂; d) CO, Pd(OAc)₂, KOAc, dppf, DMSO.

Compounds 1-Scheme 2 (e.g., **2-1**), are synthesized by methods known to the art. Alternatively, the bromo can be replaced by iodo, trifluoromethanesulfonyloxy or a group that can be converted to bromo, iodo or trifluoromethanesufonyloxy. Compounds **2-1** are converted to the N-formyl compounds **2-2** by treatment with a suitable reagent such as formic acid, an ester of formic acid, or acetic-formic anhydride in a suitable solvent at a suitable temperature. Compounds **2-2** are converted to the cyclic imines **2-3** by treatment with a suitable agent such as a mixture of polyphosphoric acid and phosphoryl chloride at a suitable temperature. Compounds **2-3** are converted to the acetates **2-4** by treatment with a suitable reagent such as the t-butyldimethylsilyl ketal acetal of methyl acetate in the presence of trimethylsilyl cyanide and a suitable catalyst such as di-µ-chloro-bis(1,5-cyclooctadiene)-dirhodium ([Rh(COD)Cl]2) in a suitable solvent such as dichloromethane using the general procedure in *Bull. Chem. Soc Jpn.* **1990**, *63*, 3122-3131. Alternatively, a Reformatsky reagent may be employed using the general procedure in *Bull. Soc. Chim. Fr.* **1973,** 1668. Alternatively, acetic anhydride in acetic acid may be employed using the general procedure in *J. Am. Chem. Soc.* **1950,** *72*, 3874.

Compounds **2-4** are converted to the carboxylic acids **2-5** according to the general method described in Scheme 1.

Compounds of formula (IV) where U is NR'CO may also be accessed directly from compounds **1-9** or **2-5** by treatment with CO, a primary or secondary amine and a palladium catalyst using the general method of *J. Org. Chem.* **1974,** *39*, 3327.

The simple tri-substituted benzene starting materials are commercially available or prepared by routine methods well known in the art.

Coupling methods to form amide bonds are generally well known to the art. The methods of peptide synthesis generally set forth by Bodansky *et al*., THE PRACTICE OF PEPTIDE SYNTHESIS, Springer-Verlag, Berlin, 1984, Ali *et al.* in *J. Med. Chem*., 29, 984 (1986) and *J. Med. Chem*., 30, 2291 (1987) are generally illustrative of the technique and are incorporated herein by reference. Coupling reagents as used herein denote reagents which may be used to form amide bonds. Typical coupling methods employ carbodiimides, activated anhydrides and esters and acyl halides. Reagents such as EDC, DCC, DPPA, BOP reagent, HOBt, N-hydroxysuccinimide and oxalyl chloride are typical.

Typically, the amine or aniline is coupled via its free amino group to an appropriate carboxylic acid substrate using a suitable carbodiimide coupling agent, such as N,N' dicyclohexyl carbodiimide (DCC), optionally in the presence of catalysts such as 1-hydroxybenzotriazole (HOBt) and dimethylamino pyridine (DMAP). Other methods, such as the formation of activated esters, anhydrides or acid halides, of the free carboxyl of a suitably protected acid substrate, and subsequent reaction with the free amine of a suitably protected amine, optionally in the presence of a base, are also suitable. For example, a protected Boc-amino acid or Cbz-amidino benzoic acid is treated in an anhydrous solvent, such as methylene chloride or tetrahydrofuran(THF), in the presence of a base, such as N-methyl morpholine, DMAP or a trialkylamine, with isobutyl chloroformate to form the "activated anhydride", which is subsequently reacted with the free amine of a second protected amino acid or aniline.

Compounds such as **1-10** are converted to compounds of formula (IV) by a coupling reaction, such as an amide coupling reaction as in Scheme 3. 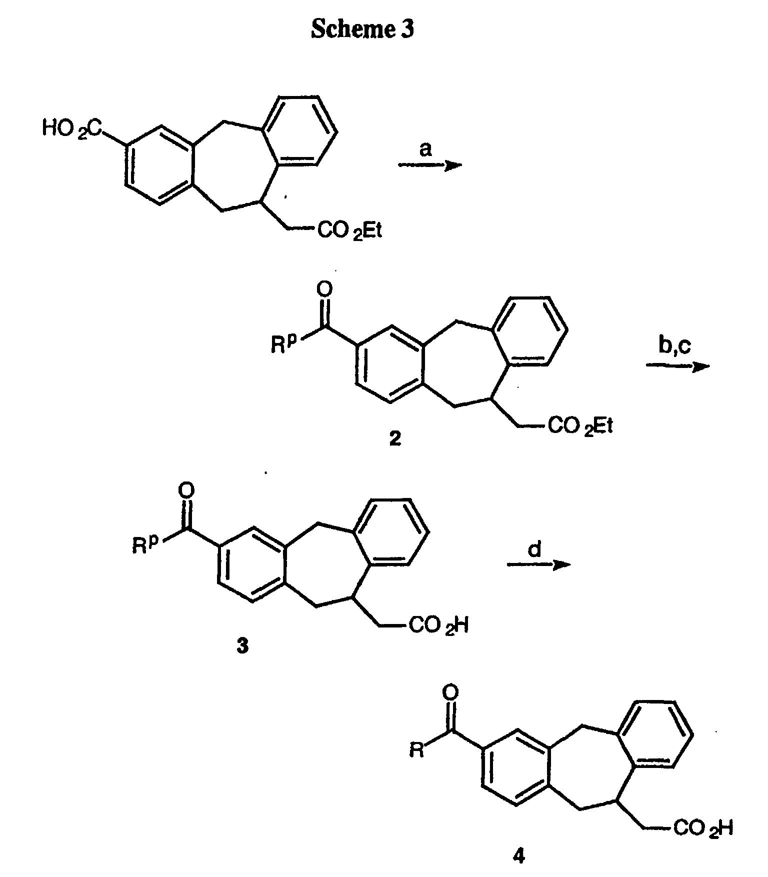 a) 1-BOC-4,4'-bipiperidine [R^{p}], EDC, HOBt · H₂O, (i-Pr)₂NEt, DMF; b) 1.0 N LiOH, THF, H₂O; c) 1.0 N HCI, H₂O; d) TFA, CH₂Cl₂ [R = 1-(4,4'-bipiperidine)].

Ethyl (±)-10,11-dihydro-3-carboxy-5H-dibenzo[a,d]cycloheptene-10-acetate (**1-10**), prepared as described in Scheme 1, is converted to an activated form of the carboxylic acid using, for example, EDC and HOBt, or SOCl₂, and the activated form is subsequently reacted with an appropriate amine, for instance 1-BOC-4,4'-bipiperidine or 2-(methylamino)methylbenzimidazole dihydrochloride, in a suitable solvent such as DMF, CH₂Cl₂, or CH₃CN, to afford compound 2-Scheme 3 (e.g., **3-2**). Depending on whether acid neutralization is required, an added base, such as diisopropylethylamine ((i-Pr)₂NEt) or pyridine, may be used. Many additional methods for converting a carboxylic acid to an amide are known, and can be found in standard reference books, such as "Compendium of Organic Synthetic Methods", Vol. I-VI (published by Wiley-Interscience). The ethyl ester **5-2** is hydrolyzed using aqueous base, for example, LiOH in aqueous THF or NaOH in aqueous methanol, and the intermediate carboxylate salt is acidified with a suitable acid, for instance TFA or HCl, to afford the carboxylic acid 1-3. Alternatively, the intermediate carboxylate salt can be isolated, if desired, or a carboxylate salt of the free carboxylic acid can be prepared by methods well-known to those of skill in the art. If the amine component of the amide bond-forming reaction (**1-10** to **3-2**) contains a protecting group, the protecting group can be removed either prior or subsequent to the ester hydrolysis step, using methods suitable for selective deprotection of the specific protecting group employed. Such methods are described in Green, "Protective Groups in Organic Synthesis" (published by Wiley-Interscience). For example, if the amine component contains a nitrogen group which is protected by a tert-butoxycarbonyl (BOC) group, such as in compound **3-3**, the BOC group is removed under acidic conditions, using, for instance, 4 N HCl in dioxane or trifluoroacetic acid (TFA) in CH₂Cl₂, to give the ammonium salt of **3-4**. The ammonium salt can be neutralized, if desired, by methods known to those of skill in the art.

Scheme 4 is illustrative of a carbon-carbon bond-forming coupling method which may be used to introduce a carbon-carbon triple bond, double bond or single bond by the optional application of an appropriate reducing agent (e.g., in step b). 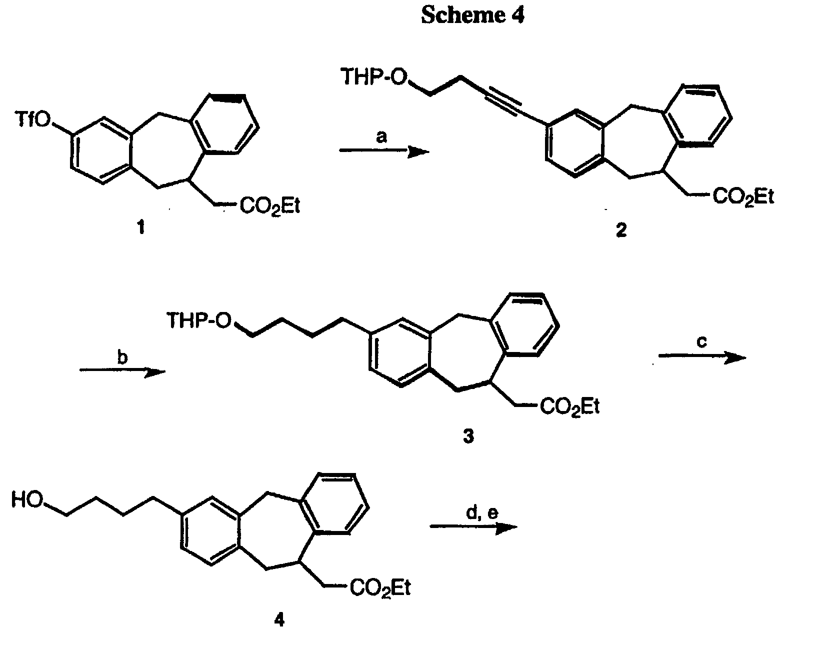 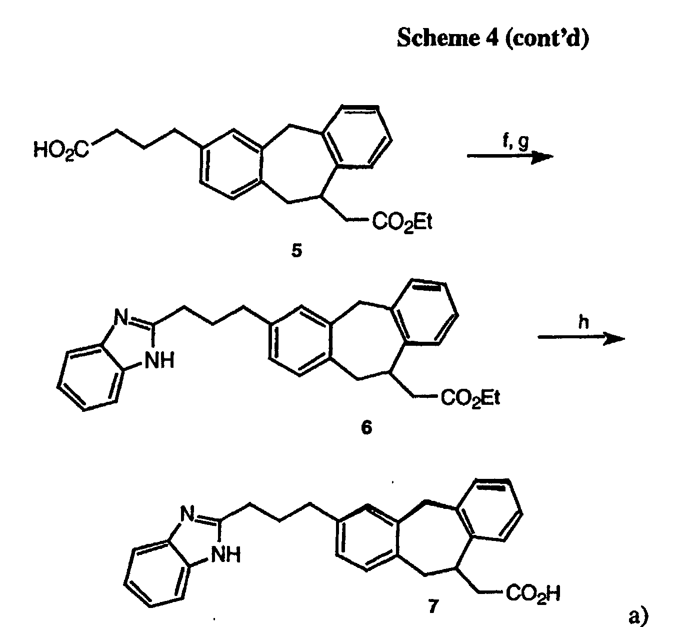 THPOCH₂CH₂CCSn(Bu)₃, (PPh₃)₂PdCl₂, LiCl, dioxane; b) H₂, 10% Pd/C, EtOAc; c) *p*-TsOH · H₂O, EtOH; d) 2,2,6,6-tetramethyloxopiperidinium chloride, CH₂Cl₂; e) NaClO₂, Na₂HPO₃, 2-methyl-2-butene, H₂O; f) isobutyl chloroformate, 4-methylmorpholine, then 1,2-phenylenediamine; g) AcOH, THF; h) 1.0 N NaOH, EtOH, then acidification.

Compound 1-Scheme 4 (**4-1**), is reacted with 4-(2-tetrahydropyranyloxy)-1-tributylstannyl-1-butyne in a Stille-type coupling reaction of aromatic triflates and organostannanes (*J. Am. Chem*. *Soc.* **1987,** *109*, 5478-5486) to afford **4-2.** The reaction is catalyzed by a palladium salt, preferably bis(triphenylphosphine)palladium (II) chloride ((PPh₃)₂PdCl₂), and is conducted in the presence of lithium chloride, in an appropriate inert solvent, generally DMF or 1,4-dioxane. Reduction of the acetylenic unit of **4-2** is accomplished under standard hydrogenation conditions which are well-known to those of skill in the art. The resulting compound, **4-3,** is deprotected under standard conditions for removal of a tetrahydropyranyl (THP) ether, to afford **4-4.** A variety of conditions for deprotection of THP ethers are described in standard reference volumes, such as Greene, "Protective Groups in Organic Synthesis" (published by Wiley-Interscience). The primary alcohol moiety of **4-4** is oxidized to the corresponding carboxylic acid **4-5** by the two-step method described by Wovkulich *(J. Org. Chem*. **1993**, *58*, 832-839). Many alternative methods for the oxidation of a primary alcohol to the corresponding carboxylic acid have been described, and can be found in such reference volumes. Conversion of the carboxylic acid of **4-5** to the benzimidazole derivative **4-6** follows the general procedures described in WO. Thus, **4-5** is initially converted to an activated form of the carboxylic acid using, for example, isobutyl chloroformate, in the presence of a suitable base, generally 4-methylmorpholine, triethylamine, or diisopropylethylamine, in an inert solvent such as THF or CH₂Cl₂. The activated form is subsequently reacted with an excess of an appropriate 1,2-diaminoaromatic derivative, for instance 1,2-phenylenediamine, to afford the corresponding mono-amide. The mono-amide is then cyclized under standard conditions, for instance acetic acid in refluxing THF, to afford **4-6**. The ethyl ester of **4-6** is hydrolyzed using aqueous base, for example, LiOH in aqueous THF or NaOH in aqueous methanol or ethanol, and the intermediate carboxylate salt is acidified with a suitable acid, for instance TFA or HCI, to afford the carboxylic acid **4-7**. Alternatively, the intermediate carboxylate salt can be isolated, if desired, or a carboxylate salt of the free carboxylic acid can be prepared by methods well-known to those of skill in the art.

Scheme 5 is illustrative of a carbon-oxygen bond forming coupling method that may be used to form an ether linkage. Similar coupling methods may also be used to form sulfide and amine linkages. 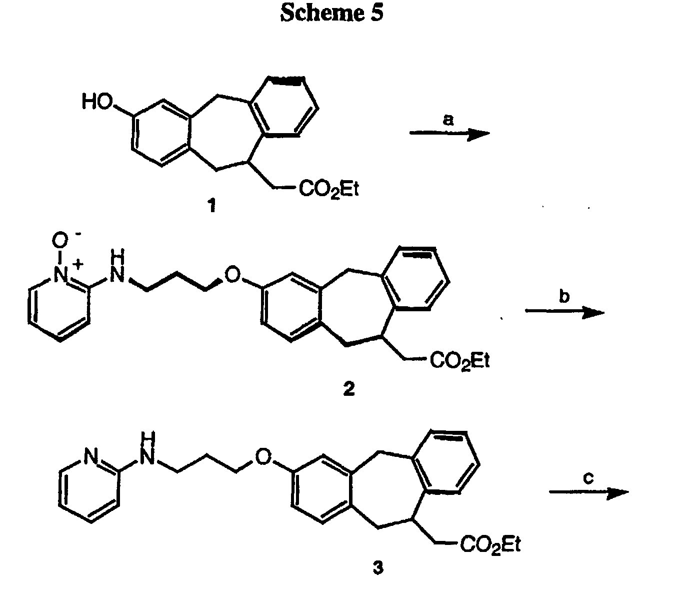 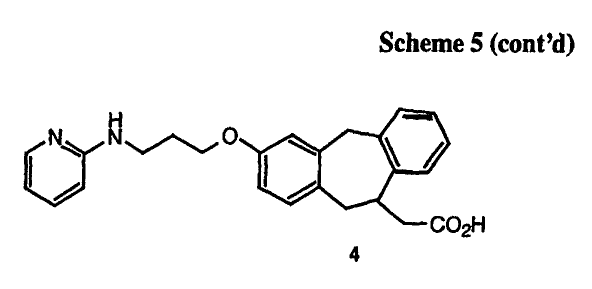 a) 2-[(3-hydroxy-1-propyl)amino]pyridine-N-oxide, DEAD, (Ph)₃P, DMF; b) cyclohexene, 10% Pd/C, 2-propanol; c) 1.0 N NaOH, EtOH, then acidification.

Compound 1 of Scheme 5 (**5-1**) is reacted with 2-[(3-hydroxy-1-propyl)ammo]pyridine-N-oxide in a Mitsunobu-type coupling reaction *(Organic Reactions* **1992,** *42*, 335-656; *Synthesis* **1981,** 1-28) to afford **5-2.** The reaction is mediated by the complex formed between diethyl azodicarboxylate and triphenylphosphine, and is conducted in an aprotic solvent, for instance THF, CH₂Cl₂, or DMF. The pyridine-N-oxide moiety of **5-2** is reduced to the corresponding pyridine **5-3** under transfer hydrogenation conditions using a palladium catalyst, preferably palladium metal on activated carbon, in an inert solvent, for instance methanol, ethanol, or 2-propanol. Cyclohexene, 1,4-cyclohexadiene, formic acid, and salts of formic acid, such as potassium formate or ammonium formate, are commonly used as the hydrogen transfer reagent in this type of reaction. The ethyl ester of **5-3** is saponified as described in Scheme 1 to afford **5-4**.

Acid addition salts of the compounds are prepared in a standard manner in a suitable solvent from the parent compound and an excess of an acid, such as hydrochloric, hydrobromic, hydrofluoric, sulfuric, phosphoric, acetic, trifluoroacetic, maleic, succinic or methanesulfonic. Certain of the compounds form inner salts or zwitterions which may be acceptable. Cationic salts are prepared by treating the parent compound with an excess of an alkaline reagent, such as a hydroxide, carbonate or alkoxide, containing the appropriate cation; or with an appropriate organic amine. Cations such as Li⁺, Na⁺, K⁺, Ca⁺⁺, Mg⁺⁺ and NH₄⁺ are specific examples of cations present in pharmaceutically acceptable salts.

This invention also provides a pharmaceutical composition which comprises a compound according to formula (IV) and a pharmaceutically acceptable carrier. Accordingly, the compounds of formula (IV) may be used in the manufacture of a medicament. Pharmaceutical compositions of the compounds of formula (IV) prepared as hereinbefore described may be formulated as solutions or lyophilized powders for parenteral administration. Powders may be reconstituted by addition of a suitable diluent or other pharmaceutically acceptable carrier prior to use. The liquid formulation may be a buffered, isotonic, aqueous solution. Examples of suitable diluents are normal isotonic saline solution, standard 5% dextrose in water or buffered sodium or ammonium acetate solution. Such formulation is especially suitable for parenteral administration, but may also be used for oral administration or contained in a metered dose inhaler or nebulizer for insufflation. It may be desirable to add excipients such as polyvinylpyrrolidone, gelatin, hydroxy cellulose, acacia, polyethylene glycol, mannitol, sodium chloride or sodium citrate.

Alternately, these compounds may be encapsulated, tableted or prepared in a emulsion or syrup for oral administration. Pharmaceutically acceptable solid or liquid carriers may be added to enhance or stabilize the composition, or to facilitate preparation of the composition. Solid carriers include starch, lactose, calcium sulfate dihydrate, terra alba, magnesium stearate or stearic acid, talc, pectin, acacia, agar or gelatin. Liquid carriers include syrup, peanut oil, olive oil, saline and water. The carrier may also include a sustained release material such as glyceryl monostearate or glyceryl distearate, alone or with a wax. The amount of solid carrier varies but, preferably, will be between about 20 mg to about 1 g per dosage unit. The pharmaceutical preparations are made following the conventional techniques of pharmacy involving milling, mixing, granulating, and compressing, when necessary, for tablet forms; or milling, mixing and filling for hard gelatin capsule forms. When a liquid carrier is used, the preparation will be in the form of a syrup, elixir, emulsion or an aqueous or non-aqueous suspension. Such a liquid formulation may be administered directly p.o. or filled into a soft gelatin capsule.

For rectal administration, the compounds of this invention may also be combined with excipients such as cocoa butter, glycerin, gelatin or polyethylene glycols and molded into a suppository.

The compounds described herein which are antagonists of the vitronectin receptor, are useful in the manufacture of a medicament for treating diseases wherein the underlying pathology is attributable to ligand or cell which interacts with the vitronectin receptor. For instance, these compounds are useful in the manufacture of a medicament for the treatment of diseases wherein loss of the bone matrix creates pathology. Thus, the instant compounds are useful in the manufacture of a medicament for the treatment of osteoporosis, hyperparathyroidism, Paget's disease, hypercalcemia of malignancy, osteolytic lesions produced by bone metastasis, bone loss due to immobilization or sex hormone deficiency. The compounds of this invention are also believed to have utility in the manufacture of a medicament as antitumor, antiinflammatory, anti-angiogenic and anti-metastatic agents, and be useful in the manufacture of a medicament treatment of cancer, atherosclerosis and restenosis. In particular, the compounds of this invention are useful in the manufacture of a medicament for inhibiting restenosis following angioplasty.

The compounds of this invention which inhibit fibrinogen binding provide a use in the manufacture of a medicament for inhibiting platelet aggregation and clot formation in a mammal, especially a human, which comprises the internal administration of a compound of formula (IV) and a pharmaceutically acceptable carrier. Indications for such therapy include acute myocardial infarction (AMI), deep vein thrombosis, pulmonary embolism, dissecting anurysm, transient ischemia attack (TIA), stroke and other infarct-related disorders, and unstable angina. Chronic or acute states of hyper-aggregability, such as disseminated intravascular coagulation (DIC), septicemia, surgical or infectious shock, post-operative and post-partum trauma, cardiopulmonary bypass surgery, incompatible blood transfusion, abruptio placenta, thrombotic thrombocytopenic purpura (TTP), snake venom and immune diseases, are likely to be responsive to such treatment. In addition, the compounds of this invention may be useful in the manufacture of a medicament for the prevention of metastatic conditions, the prevention or treatment of fungal or bacterial infection, inducing immunostimulation, treatment of sickle cell disease, and the prevention or treatment of diseases in which bone resorption is a factor.

This invention further provides a use in the manufacture of a medicament for inhibiting the reocclusion of an artery or vein following fibrinolytic therapy, which comprises internal administration of a compound of formula (IV) and a fibrinolytic agent. Administration of a compound of formula (IV) in fibrinolytic therapy either prevents reocclusion completely or prolongs the time to reocclusion. When used in the context of this invention the term fibrinolytic agent is intended to mean any compound, whether a natural or synthetic, product, which directly or indirectly causes the lysis of a fibrin clot. Plasminogen activators are a well known group of fibrinolytic agents. Useful plasminogen activators include, for example, anistreplase, urokinase (UK), pro-urokinase (pUK), streptokinase (SK), tissue plasminogen activator (tPA) and mutants, or variants, thereof.

The compounds of this invention may also be used *in vitro* to inhibit the aggregation of platelets in blood and blood products, *e.g.,* for storage, or for *ex vivo* manipulations such as in diagnostic or research use.

The compound is administered either orally or parenterally to the patient, in a manner such that the concentration of drug is sufficient to inhibit bone resorption, or inhibit platelet aggregation or other such indication. The pharmaceutical composition containing the compound is administered at an oral dose of between about 0.1 to about 50 mg/kg in a manner consistent with the condition of the patient. Preferably the oral dose would be about 0.5 to about 20 mg/kg. For acute therapy, parenteral administration is preferred. An intravenous infusion of the compound in 5% dextrose in water or normal saline, or a similar formulation with suitable excipients, is most effective, although an intramuscular bolus injection is also useful. Typically, the parenteral dose will be about 0.01 to about 100 mg/kg; preferably between 0.1 and 20 mg/kg. The compounds are administered one to four times daily at a level to achieve a total daily dose of about 0.4 to about 400 mg/kg/day. The precise level and method by which the compounds are administered is readily determined by one routinely skilled in the art by comparing the blood level of the agent to the concentration required to have a therapeutic effect.

The compounds may be tested in one of several biological assays to determine the concentration of compound which is required to have a given pharmacological effect.

### INHIBITION OF VITRONECTIN BINDING

*Solid-Phase [*^{*3*}*H]-SK&F-107260 Binding to α*_{*v*}*β*_{*3*}*:* Human placenta or human platelet αᵥβ₃ (0.1-0.3 mg/mL) in buffer T (containing 2 mM CaCl₂ and 1% octylglucoside) was diluted with buffer T containing 1 mM CaCl₂, 1 mM MnCl₂, 1 mM MgCl₂ (buffer A) and 0.05% NaN₃, and then immediately added to 96-well ELISA plates (Corning, New York, NY) at 0.1 mL per well. 0.1 - 0.2 µg of αᵥβ₃ was added per well. The plates were incubated overnight at 4°C. At the time of the experiment, the wells were washed once with buffer A and were incubated with 0.1 mL of 3.5% bovine serum albumin in the same buffer for 1 hr at room temperature. Following incubation the wells were aspirated completely and washed twice with 0.2 mL buffer A.

Compounds were dissolved in 100% DMSO to give a 2 mM stock solution, which was diluted with binding buffer (15 mM Tris-HCl (pH 7.4), 100 mM NaCl, 1 mM CaCl₂, 1 mM MnCl₂, 1 mM MgCl₂) to a final compound concentration of 100 µM. This solution is then diluted to the required final compound concentration. Various concentrations of unlabeled antagonists (0.001 - 100 µM) were added to the wells in triplicates, followed by the addition of 5.0 nM of [³H]-SK&F-107260 (65 - 86 Ci/mmol).

The plates were incubated for 1 hr at room temperature. Following incubation the wells were aspirated completely and washed once with 0.2 mL of ice cold buffer A in a well-to-well fashion. The receptors were solubilized with 0.1 mL of 1% SDS and the bound [³H]-SK&F-107260 was determined by liquid scintillation counting with the addition of 3 mL Ready Safe in a Beckman LS Liquid Scintillation Counter, with 40% efficiency. Nonspecific binding of [³H]-SK&F-107260 was determined in the presence of 2 µM SK&F-107260 and was consistently less than 1% of total radioligand input. The IC₅₀ (concentration of the antagonist to inhibit 50% binding of [³H]-SK&F-107260) was determined by a nonlinear, least squares curve-fitting routine, which was modified from the LUNDON-2 program. The Kᵢ (dissociation constant of the antagonist) was calculated according to the equation: Kᵢ = IC₅₀/(1 + L/K_{d}), where L and K_{d} were the concentration and the dissociation constant of [³H]-SK&F-107260, respectively.

Compounds of the present invention inhibit vitronectin binding to SK&F 107260 in the concentration range of 0.1 to 25 micromolar. Preferred compounds inhibit vitronectin binding at a concentration of less than 1 micromolar.

Compounds of this invention are also tested for *in vitro* and *in vivo* bone resorption in assays standard in the art for evaluating inhibition of bone formation, such as the pit formation assay disclosed in EP 528 587, which may also be performed using human osteoclasts in place of rat osteoclasts, and the ovarectomized rat model, described by Wronski *et al., Cells and Materials* **1991,** Sup. 1, 69-74.

### PARATHYROIDECTOMIZED RAT MODEL

Each experimental group consists of 5-6 male Sprague-Dawley rats. The rats are parathyroidectomized (by the vendor, Taconic Farms) 7 days prior to use. Twenty four hours prior to use, circulating ionized calcium levels are measured in whole blood immediately after it has been withdrawn by tail venipuncture into heparinized tubes. Rats are included if ionized Ca level (measured with a Ciba-Corning model 634 calcium pH analyzer) is •1.2 mM/L. The rats are then put on a diet of calcium-free chow and deionized water. At the start of the experiment the rats weigh approximately 100g. Baseline Ca levels are measured and the rats are administered control vehicle (saline) or compound (dissolved in saline) as a single intravenous (tail vein) bolus injection followed immediately by a single subcutaneous injection of either human parathyroid hormone 1-34 peptide (hPTH1-34, dose 0.2mg/kg in saline/0.1% bovine serum albumen, Bachem, Ca) or the PTH vehicle. The calcemic response to PTH (and any effect of compound on this response) is measured 2h after compound/PTH administration.

### RAT ULNA DRIFT MODEL

Each experimental group consists of 8-10 male Sprague-Dawley or Wistar rats of approximately 30-40g body weight at the start of the experiment. The agent being tested is administered by an appropriate route as single or multiple daily doses for a period of seven days. Prior to administration of the first dose, the rats are given a single dose of a fluorescent marker (tetracycline 25mg/kg, or calcein 10mg/kg) that labels the position of bone forming surfaces at that point in time. After dosing of compound has been completed, the rats are killed and both forelimbs are removed at the elbow, the foot is removed at the ankle and the skin removed. The sample is frozen and mounted vertically on a microtome chuck. Cross sections of the midshaft region of the ulna are cut in the cryostat. The rate of bone resorption is measured morphometrically in the medial-dorsal portion of the cortical bone. The measurement is done as follows: the amount of bone resorbed at the periosteal surface is equal to the distance by which the periosteal surface has advanced towards the fluorescent label which had been incorporated at the endosteal bone formation surface on day zero; this distance is calculated by subtracting the width of bone between the label and the periosteal surface on day 7 from the width on day zero; the resorption rate in microns per day is calculated by dividing the result by 7.

### HUMAN OSTEOCLAST RESORPTION ASSAY ("PIT ASSAY")

- Aliquots of osteoclastoma-derived cell suspensions are removed from liquid nitrogen storage, warmed rapidly at 37°C and washed x1 in RPMI-1640 medium by centrifugation (1000rpm, 5 mins at 4°C).
- Aspirate the medium and replace it with murine anti-HLA-DR antibody, diluted 1:3 in RPMI-1640 medium. Incubate for 30 mins on ice and mix the cell suspension frequently.
- The cells are washed x2 with cold RPMI-1640 by centrifugation (1000rpm, 5 mins at 4°C) and the cells are transferred to a sterile 15 ml centrifuge tube. The number of mononuclear cells are enumerated in an improved Neubauer counting chamber.
- Sufficient magnetic beads (5 / mononuclear cell), coated with goat anti-mouse IgG, are removed from their stock bottle and placed into 5 ml of fresh medium (this washes away the toxic azide preservative). The medium is removed by immobilizing the beads on a magnet and is replaced with fresh medium.
- The beads are mixed with the cells and the suspension is incubated for 30 mins on ice. The suspension is mixed frequently.
- The bead-coated cells are immobilized on a magnet and the remaining cells (osteoclast-rich fraction) are decanted into a sterile 50 ml centrifuge tube.
- Fresh medium is added to the bead-coated cells to dislodge any trapped osteoclasts. This wash process is repeated x10. The bead-coated cells are discarded.
- The osteoclasts are enumerated in a counting chamber, using a large-bore disposable plastic pasteur to charge the chamber with the sample.
- The cells are pelleted by centrifugation and the density of osteoclasts adjusted to 1.5x10⁴/ml in EMEM medium, supplemented with 10% fetal calf serum and 1.7g/litre of sodium bicarbonate.
- 3ml aliquots of the cell suspension ( per treatment) are decanted into 15ml centrifuge tubes. The cells are pelleted by centrifugation.
- To each tube 3ml of the appropriate treatment are added (diluted to 50 µM in the EMEM medium). Also included are appropriate vehicle controls, a positive control (87MEM1 diluted to 100 µg/ml) and an isotype control (IgG2a diluted to 100 µg/ml). Incubate at 37°C for 30 mins.
- 0.5ml aliquots of the cells are seeded onto sterile dentine slices in a 48-well plate and incubated at 37°C for 2 hours. Each treatment is screened in quadruplicate.
- The slices are washed in six changes of warm PBS (10 ml / well in a 6-well plate) and then placed into fresh treatment or control. Incubate at 37°C for 48 hours.
   **tartrate resistant acid phosphatase (trap) procedure** (selective stain for cells of the osteoclast lineage).
- The slices are washed in phosphate buffered saline and fixed in 2% gluteraldehyde (in 0.2M sodium cacodylate) for 5 mins.
- They are washed in water and incubated in TRAP buffer for 5 mins at 37°C.
- Following a wash in cold water they are incubated in cold acetate buffer / fast red garnet for 5 mins at 4°C.
- Excess buffer is aspirated, and the slices are air dried following a wash in water.
- The TRAP positive osteoclasts are enumerated by bright-field microscopy and are then removed from the surface of the dentine by sonication.
- Pit volumes are determined using the Nikon/Lasertec ILM21W confocal microscope.

### INHIBITION OF RGD-MEDIATED α_{IIb}β₃ BINDING

### Purification of α_{IIb}β₃

Ten units of outdated, washed human platelets (obtained from Red Cross) were lyzed by gentle stirring in 3% octylglucoside, 20 mM Tris-HCl, pH 7.4, 140 mM NaCl, 2 mM CaCl₂ at 4°C for 2 h. The lysate was centrifuged at 100,000g for 1 h. The supernatant obtained was applied to a 5 mL lentil lectin sepharose 4B column (E.Y. Labs) preequilibrated with 20 mM Tris-HCl, pH 7.4, 100 mM NaCI, 2 mM CaCl₂, 1% octylglucoside (buffer A). After 2 h incubation, the column was washed with 50 mL cold buffer A. The lectin-retained α_{IIb}β₃ was eluted with buffer A containing 10% dextrose. All procedures were performed at 4°C. The α_{IIb}β₃ obtained was >95% pure as shown by SDS polyacrylamide gel electrophoresis.

### Incorporation of α_{IIb}β₃ in Liposomes

A mixture of phosphatidylserine (70%) and phosphatidylcholine (30%) (Avanti Polar Lipids) were dried to the walls of a glass tube under a stream of nitrogen. Purified α_{IIb}β₃ was diluted to a final concentration of 0.5 mg/mL and mixed with the phospholipids in a protein:phospholipid ratio of 1:3 (w:w). The mixture was resuspended and sonicated in a bath sonicator for 5 min. The mixture was then dialyzed overnight using 12,000-14,000 molecular weight cutoff dialysis tubing against a 1000-fold excess of 50 mM Tris-HCl, pH 7.4, 100 mM NaCl, 2 mM CaCl2 (with 2 changes). The α_{IIb}β₃-containing liposomes wee centrifuged at 12,000g for 15 min and resuspended in the dialysis buffer at a final protein concentration of approximately 1 mg/mL. The liposomes were stored at -70°C until needed.

### Competitive Binding to α_{IIb}β₃

The binding to the fibrinogen receptor (α_{IIb}β₃) was assayed by an indirect competitive binding method using [³H]-SK&F-107260 as an RGD-type ligand. The binding assay was performed in a 96-well filtration plate assembly (Millipore Corporation, Bedford, MA) using 0.22 um hydrophilic durapore membranes. The wells were precoated with 0.2 mL of 10 µg/mL polylysine (Sigma Chemical Co., St. Louis, MO.) at room temperature for 1 h to block nonspecific binding. Various concentrations of unlabeled benzodiazepines were added to the wells in quadruplicate. [³H]-SK&F-107260 was applied to each well at a final concentration of 4.5 nM, followed by the addition of 1 µg of the purified platelet α_{IIb}β₃-containing liposomes. The mixtures were incubated for 1 h at room temperature. The α_{IIb}β₃-bound [³H]-SK&F-107260 was separated from the unbound by filtration using a Millipore filtration manifold, followed by washing with ice-cold buffer (2 times, each 0.2 mL). Bound radioactivity remaining on the filters was counted in 1.5 mL Ready Solve (Beckman Instruments, Fullerton, CA) in a Beckman Liquid Scintillation Counter (Model LS6800), with 40% efficiency. Nonspecific binding was determined in the presence of 2 µM unlabeled SK&F-107260 and was consistently less than 0.14% of the total radioactivity added to the samples. All data points are the mean of quadruplicate determinations.

Competition binding data were analyzed by a nonlinear least-squares curve fitting procedure. This method provides the IC50 of the antagonists (concentration of the antagonist which inhibits specific binding of [³H]-SK&F-107260 by 50% at equilibrium). The IC50 is related to the equilibrium dissociation constant (Ki) of the antagonist based on the Cheng and Prusoff equation: Ki = IC50/(1+L/Kd), where L is the concentration of [3H]-SK&F-107260 used in the competitive binding assay (4.5 nM), and Kd is the dissociation constant of [3H]-SK&F-107260 which is 4.5 nM as determined by Scatchard analysis.

Inhibition of platelet aggregation may be measured by the method described in WO 93/00095 (PCT/US/92/05463). *In vivo* thrombus formation is demonstrated by recording the systemic and hemodynamic effects of infusion of the peptides into anesthetized dogs according to the methods described in Aiken et al., *Prostaglandins*, 19, 620 (1980).

Preferred compounds of this invention have an affinity for the vitronectin receptor relative to the fibrinogen receptor, or for the fibrinogen receptor relative to the vitronectin receptor, of greater than 5:1. More preferred compounds have a ratio of activity of greater than 10:1. The most preferred compounds have a selectivity of greater than 100:1. The comparative results of the enhanced binding of the compounds of this invention to the vitronectin receptor relative to the fibrinogen receptor are given in Table 1 below:

| Compound (Ex. #) | Ki/α_{IIb}β₃ (µM) | Ki/α_{V}β₃(µM) |
|---|---|---|
| 1 | >50 | 0.023 |
| 2 | 0.009 | 15 |

### Vascular smooth muscle cell migration assay

The compounds of the instant invention were tested for their ability to inhibit the migration and proliferation of smooth muscle tissue in an artery or vein in order to assess their ability to prevent restenosis of an artery, such as that which typically occurs following angioplasty.

Rat or human aortic smooth muscle cells were used. The cell migration was monitored in a Transwell cell culture chamber by using a polycarbonate membrane with pores of 8 um (Costar). The lower surface of the filter was coated with vitronectin. Cells were suspended in DMEM supplemented with 0.2% bovine serum albumin at a concentration of 2.5 - 5.0 x 10⁶ cells/mL, and were pretreated with test compound at various concentrations for 20 min at 20°C. The solvent alone was used as control. 0.2 mL of the cell suspension was placed in the upper compartment of the chamber. The lower compartment contained 0.6 mL of DMEM supplemented with 0.2% bovine serum albumin. Incubation was carried out at 37°C in an atmosphere of 95% air/5% CO₂ for 24 hr. After incubation, the non-migrated cells on the upper surface of the filter were removed by gentle scraping. The filter was then fixed in methanol and stained with 10% Giemsa stain. Migration was measured either by a) counting the number of cells that had migrated to the lower surface of the filter or by b) extracting the stained cells with 10% acetic acid followed by determining the absorbance at 600 nM.

### General

Nuclear magnetic resonance spectra were recorded at either 250 or 400 MHz using, respectively, a Bruker AM 250 or Bruker AC 400 spectrometer. CDCl₃ is deuteriochloroform, DMSO-d₆ is hexadeuteriodimethylsulfoxide, and CD₃OD is tetradeuteriomethanol. Chemical shifts are reported in parts per million (δ) downfield from the internal standard tetramethylsilane. Abbreviations for NMR data are as follows: s = singlet, d = doublet, t = triplet, q = quartet, m = multiplet, dd = doublet of doublets, dt = doublet of triplets, app = apparent, br = broad. J indicates the NMR coupling constant measured in Hertz. Continuous wave infrared (IR) spectra were recorded on a Perkin-Elmer 683 infrared spectrometer, and Fourier transform infrared (FTIR) spectra were recorded on a Nicolet Impact 400 D infrared spectrometer. IR and FTIR spectra were recorded in transmission mode, and band positions are reported in inverse wavenumbers (cm⁻¹). Mass spectra were taken on either VG 70 FE, PE Syx API III, or VG ZAB HF instruments, using fast atom bombardment (FAB) or electrospray (ES) ionization techniques. Elemental analyses were obtained using a Perkin-Elmer 240C elemental analyzer. Melting points were taken on a Thomas-Hoover melting point apparatus and are uncorrected. All temperatures are reported in degrees Celsius.

Analtech Silica Gel GF and E. Merck Silica Gel 60 F-254 thin layer plates were used for thin layer chromatography. Both flash and gravity chromatography were carried out on E. Merck Kieselgel 60 (230-400 mesh) silica gel. Analytical and preparative HPLC were carried out on Rainin or Beckman chromatographs. ODS refers to an octadecylsilyl derivatized silica gel chromatographic support. 5 µ Apex-ODS indicates an octadecylsilyl derivatized silica gel chromatographic support having a nominal particle size of 5 µ, made by Jones Chromatography, Littleton, Colorado. YMC ODS-AQ® is an ODS chromatographic support and is a registered trademark of YMC Co. Ltd., Kyoto, Japan. PRP-1® is a polymeric (styrene-divinylbenzene) chromatographic support, and is a registered trademark of Hamilton Co., Reno, Nevada. Celite® is a filter aid composed of acid-washed diatomaceous silica, and is a registered trademark of Manville Corp., Denver, Colorado.

### Preparation 1

### Preparation of ethyl (±)-10,11-dihydro-3-carboxy-5H-dibenzo[a,d]cycloheptene-10-acetate

### a) 3-Benzyl-4-(trifluoromethanesulfonyloxy)anisole

Trifluoromethanesulfonic anhydride (10.0 mL, 60 mmol) was added over 3 min to a solution of 2-benzyl-4-methoxyphenol (10.71 g, 50 mmol; prepared according to *J. Am. Chem. Soc*. **1949,** *71*, 64) and anhydrous 2,6-lutidine (12.0 mL, 100 mmol) in anhydrous CH₂Cl₂ (250 mL) at -78°C under argon. The reaction was stirred at -78°C for 0.5 h, then was warmed to RT. After 1 h, the reaction was diluted with hexanes (250 mL) and washed sequentially with 1.0 N HCl (2 x 100 mL), 1.0 N NaOH (2 x 50 mL), H₂O (100 mL) and brine (50 mL). Drying (Na₂SO₄), concentration, and silica gel chromatography (10% EtOAc/hexanes) gave the title compound as a light yellow solid (16.65 g, 96%): TLC R_{f} 0.51 (10% EtOAc/hexanes); ¹H NMR (250 MHz, CDCl₃) δ 7.10-7.40 (m, 6H), 6.77 (dd, J = 9.0, 3.1 Hz, 1H), 6.66 (d, J = 3.1 Hz, 1H), 4.03 (s, 2H), 3.73 (s, 3H); FTIR (CCl₄) 1492, 1423, 1405, 1249, 1216, 1161, 1144, 1039, 869 cm⁻¹; MS (ES) m/e 369 (M+Na)⁺, 364.0 (M+NH₄)⁺, 347.0 (M +H)⁺.

### b) 4-Allyl-3-benzylanisole

LiCl (3.08 g, 72.8 mmol) in a roundbottom flask was flame-dried in high vacuum, and the system was allowed to cool to RT under argon. 3-Benzyl-4-(trifluoromethanesulfonyloxy)anisole (Preparation 1(a)) (21.0 g, 60.6 mmol), bis(triphenylphosphine)palladium(II) chloride (2.13 g, 3.0 mmol), anhydrous DMF (150 mL), and allyltributyltin (22.6 mL, 72.8 mmol) were added, and the mixture was purged with argon through three evacuation/argon flush cycles. The mixture was heated in an oil bath preset at 95°C, affording a yellow, homogeneous solution. After 1.5 h, the dark mixture was concentrated on the rotavap (high vacuum), and the residue was reconcentrated from xylenes. The resulting residue was taken up in Et₂O (120 mL) and stirred briskly with 10% KF (120 mL) for 0.5 h. The layers were separated, and the aqueous layer was extracted with Et₂O (2 x 120 mL). The combined organics were filtered through celite® to remove the insoluble solids, and the filtrate was washed sequentially with H₂O (60 mL) and brine (60 mL). Drying (MgSO₄) and concentration left a cloudy, yellow oil. Chromatography (silica gel, 5% EtOAc/hexanes) gave the title compound as a light yellow oil (14.21 g, 98%): TLC R_{f} (5% EtOAc/hexanes) 0.51; ¹H NMR (250 MHz, CDCl₃) δ 7.03-7.31 (m, 6H), 6.74 (dd, J = 8.3, 2.7 Hz, 1H), 6.66 (d, J = 2.7 Hz, 1H), 5.79-5.98 (m, 1H), 4.89-5.07 (m, 2H), 3.97 (s, 2H), 3.75 (s, 3H), 3.21-3.33 (m, 2H); FTIR (CCl₄) 1610, 1496, 1256, 1046, 914 cm⁻¹; MS (ES) m/e 239.2 (M +H)⁺.

### c) 2-Benzyl-4-methoxyphenylacetic acid

A solution of H₅IO₆ (23.83 g, 104.5 mmol) in H₂O (56 mL) was added to a solution of 4-allyl-3-benzylanisole (Preparation 1(b)) (5.30 g, 22.24 mmol) in CCl₄ (28 mL) and CH₃CN (28 mL), and the well-stirred mixture was cooled thoroughly to 0°C. RuCl₃ (231 mg, 1.11 mmol) was added, and the reaction was stirred briskly at 0°C for 4 h, then at RT for 45 min. The mixture was filtered through celite®, and the filter pad was washed with CH₂Cl₂ (120 mL) then with H₂O (120 mL). The layers were separated and the aqueous layer was extracted with CH₂Cl₂ (3 x 120 mL). Drying (Na₂SO₄) and concentration left a brown oil. This was partitioned between Et₂O (90 mL) and 0.25 N NaOH (90 mL), and the layers were separated. The Et₂O layer was extracted with 0.25 N NaOH (2 x 10 mL), and the combined aqueous layers were acidified (pH 2) with conc. HCI. CH₂Cl₂ extraction, drying (Na₂SO₄), and concentration gave the title compound as a yellow oil which solidified to a yellow solid (4.19 g, 74%): ¹H NMR (250 MHz, CDCl₃) δ 7.05-7.35 (m, 6H), 6.77 (dd, J = 8.3, 2.7 Hz, 1H), 6.71 (d, J = 2.7 Hz, 1H), 4.00 (s, 2H), 3.76 (s, 3H), 3.54 (s, 2H); FTIR (CCl₄) 2300-3500 (broad), 1710, 1611, 1502, 1496, 1285, 1257, 1045 cm⁻¹; MS (ES) m/e 279.0 (M+Na)⁺, 274.0 (M+NH₄)⁺, 257.0 (M +H)⁺.

### d) 3-Methoxy-5H-dibenzo[a,d]cycloheptene-10(11H)-one

Finely powdered 2-benzyl-4-methoxyphenylacetic acid (Preparation 1(c)) (3.26 g, 12.72 mmol) was added to well-stirred polyphosphoric acid (165 g) at 100-110°C. After 15 min, the reaction was poured onto ice (330 g). Et₂O (330 mL) was added, and the mixture was stirred briskly for 15 min. The layers were separated, and the aqueous layer was extracted with Et₂O (330 mL). The combined organic layers were washed with 5% NaHCO₃ (2 x 80 mL) then with brine (80 mL), dried (MgSO₄), and concentrated. The residue was reconcentrated from toluene, then was chromatographed (silica gel, 20% EtOAc/hexanes). The title compound was obtained as a yellow solid (1.44 g, 48%): TLC R_{f} (20% EtOAc/hexanes) 0.46; ¹H NMR (250 MHz, CDCl₃) δ 8.07-8.15 (m, 1H), 7.39-7.49 (m, 1H), 7.25-7.48 (m, 2H), 7.19 (d, J = 8.3 Hz, 1H), 6.86 (d, J = 2.6 Hz, 1H), 6.71 (dd, J = 8.3, 2.6 Hz, 1H), 4.21 (s, 2H), 4.11 (s, 2H), 3.77 (s, 3H); FTIR (CCl₄) 1680, 1501, 1282, 1270 cm⁻¹; MS (ES) m/e 261 (M+Na)⁺, 256.0 (M+NH₄)⁺, 239.0 (M +H)⁺.

### e) Ethyl (±)-10,11-dihydro-10-hydroxy-3-methoxy-5H-dibenzo[a,d]cycloheptene-10-acetate

Anhydrous EtOAc (0.58 mL, 6.6 mmol) was added dropwise to a solution of lithium bis(trimethylsilyl)amide (1.0 M in THF, 6 mL, 6 mmol) in dry THF (24 mL) in a flame-dried flask at -78°C under argon. The yellow solution was stirred at -78°C for 0.5 h, then a solution of 3-methoxy-5H-dibenzo[a,d]cycloheptene-10(11H)-one (Preparation 1(d)) (715 mg, 3 mmol) in dry THF (3 mL) was added dropwise over 3 min. Additional dry THF (0.4 mL) was used in transfer. After 0.5 h at -78°C, the reaction was quenched with saturated NH₄Cl (15 mL), warmed to RT, and extracted with EtOAc (2 x 30 mL). Drying (MgSO₄), concentration, and chromatography (silica gel, 10% EtOAc/hexanes (400 mL), then 20% EtOAc/hexanes) gave recovered 3-methoxy-5H-dibenzo[a,d]cycloheptene-10(11H)-one (305.4 mg, 43%) as a yellow solid, followed by the title compound as a light yellow oil (531.9 mg, 54%): TLC R_{f} 0.37 (20% EtOAc/hexanes); ¹H NMR (250 MHz, CDCl₃) δ 7.63 (d, J = 7.7 Hz, 1H), 7.00-7.30 (m, 4H), 6.80 (d, J = 2.6 Hz, 1H), 6.69 (dd, J = 8.2, 2.6 Hz, 1H), 3.95-4.35 (m, 2H), 4.07 (s, 2H), 3.76 (s, 3H), 3.68 (s, 1H), 3.64 (d, J = 14.2 Hz, 1H), 3.35 (d, J = 14.2 Hz, 1H), 2.79 (d, J = 16.0 Hz, 1H), 2.66 (d, J = 16.0 Hz, 1H), 1.22 (t, J = 7.2 Hz, 3H); FTIR (CCl₄) 3580 (sharp), 3509 (broad), 1735, 1715, 1503, 1261, 1198, 1156, 1044 cm⁻¹; MS (ES) m/e 675.2 (2M+Na)⁺, 653.2 (2M +H)⁺.

### f) Ethyl (±)-10,11-dihydro-3-methoxy-5H-dibenzo[a,d]cycloheptene-10-acetate

10% Pd/C (242 mg, 0.23 mmol) was added to a solution of ethyl (±)-10,11-dihydro-10-hydroxy-3-methoxy-5H-dibenzo[a,d]cycloheptene-10-acetate (Preparation 1(e)) (741.1 mg, 2.27 mmol) and conc. HCI (0.19 mL, 2.27 mmol) in glacial AcOH (23 mL), and the mixture was shaken on a Parr apparatus at RT under H₂ (50 psi). After 6 h, the reaction was filtered through celite®, and the filter pad was washed with EtOAc. The filtrate was concentrated, and the residue was reconcentrated from toluene. The resulting faintly yellow, oily residue was chromatographed (silica gel, 20% EtOAc/hexanes) to afford the title compound as a colorless oil (643.6 mg, 91%): TLC R_{f} 0.57 (20% EtOAc/hexanes); ¹H NMR (250 MHz, CDCl₃) δ 7.05-7.22 (m, 4H), 7.01 (d, J = 8.2 Hz, 1H), 6.76 (d, J = 2.7 Hz, 1H), 6.67 (dd, J = 8.2, 2.7 Hz, 1H), 4.30 (d, J = 15.0 Hz, 1H), 4.11-4.25 (m, 2H), 3.85 (d, J = 15.0 Hz, 1H), 3.70-3.90 (m, 1H), 3.77 (s, 3H), 3.31 (dd, J = 15.0, 4.1 Hz, 1H), 2.93 (dd, J = 15.0, 9.2 Hz, 1H), 2.64 (dd, J = 15.6, 5.0 Hz, 1H), 2.52 (dd, J = 15.6, 9.3 Hz, 1H), 1.27 (t, J = 7.1 Hz, 3H); FTIR (CCl₄) 1734, 1611, 1504, 1285, 1263, 1155, 1044 cm⁻¹; MS (ES) m/e 333.0 (M+Na)⁺, 328.0 (M+NH₄)⁺, 311.0 (M +H)⁺, 265.0 (M+H-EtOH)⁺.

### g) Ethyl (±)-10,11-dihydro-3-hydroxy-5H-dibenzo[a,d]cycloheptene-10-acetate

Anhydrous AlCl₃ (1.38 g, 10.35 mmol) was added all at once to a solution of ethyl (±)-10,11-dihydro-3-methoxy-5H-dibenzo[a,d]cycloheptene-10-acetate (Preparation 1(f)) (643.6 mg, 2.07 mmol) in anhydrous CH₂Cl₂ (21 mL) at 0°C under argon. The yellow solution was warmed to RT and stirred for 3 h, then was cooled to 0°C and quenched with cold 3 N HCl (10 mL). The layers were separated, and the aqueous layer was extracted with CH₂Cl₂. The combined organic layers were dried (MgSO₄) and concentrated. Silica gel chromatography (25% EtOAc/hexanes) gave the title compound as a nearly colorless oil (611.7 mg, 100%): TLC R_{f} 0.26 (20% EtOAc/hexanes); ¹H NMR (400 MHz, CDCl₃) δ 7.03-7.22 (m, 4H), 6.93 (d, J = 8.1 Hz, 1H), 6.69 (d, J = 2.6 Hz, 1H), 6.58 (dd, J = 8.1, 2.6 Hz, 1H), 5.00 (s, 1H), 4.25 (d, J = 14.9 Hz, 1H), 4.11-4.25 (m, 2H), 3.73-3.88 (m, 1H), 3.79 (d, J = 14.9 Hz, 1H), 3.28 (dd, J = 15.0, 4.1 Hz, 1H), 2.91 (dd, J = 15.0, 9.3 Hz, 1H), 2.65 (dd, J = 15.6, 4.9 Hz, 1H), 2.53 (dd, J = 15.6, 9.5 Hz, 1H), 1.27 (t, J = 7.2 Hz, 3H); FTIR (CCl₄) 3611 (sharp), 3447 (broad), 1734, 1504, 1291, 1272, 1176, 1152 cm⁻¹; MS (ES) m/e 314.2 (M+NH₄)⁺, 297.2 (M +H)⁺.

### h) Ethyl (±)-10,11-dihydro-3-(trifluoromethanesulfonyloxy)-5H-dibenzo[a,d]cycloheptene-10-acetate

Trifluoromethanesulfonic anhydride (0.45 mL, 2.68 mmol) was added dropwise to a solution of ethyl (±)-10,11-dihydro-3-hydroxy-5H-dibenzo[a,d]cycloheptene-10-acetate (Preparation 1(g)) (611.7 mg, 2.06 mmol) and 2,6-lutidine (0.48 mL, 4.12 mmol) in anhydrous CH₂Cl₂ (10.3 mL) at -78°C under argon. After 0.5 h, the reaction was warmed to RT and stirred for 1 h. The yellow solution was diluted with Et₂O (50 mL) and washed sequentially with 1.0 N HCl (5 mL), 5% NaHCO₃ (5 mL), and brine (5 mL). Drying (MgSO₄), concentration, and silica gel chromatography (20% EtOAc/hexanes) gave the title compound as a colorless oil (808.9 mg, 92%): TLC R_{f} (20% EtOAc/hexanes) 0.58; ¹H NMR (250 MHz, CDCl₃) δ 6.98-7.30 (m, 7H), 4.35 (d, J = 15.2 Hz, 1H), 4.19 (q, J = 7.1 Hz, 2H), 3.91 (d, J = 15.2 Hz, 1H), 3.78-3.95 (m, 1H), 3.37 (dd, J = 15.2, 4.1 Hz, 1H), 3.02 (dd, J = 15.2, 9.6 Hz, 1H), 2.70 (dd, J = 15.8, 4.8 Hz, 1H), 2.53 (dd, J = 15.8, 9.6 Hz, 1H), 1.27 (t, J = 7.1 Hz, 3H); FTIR (CCl₄) 1735, 1493, 1427, 1250, 1215, 1144, 961, 856 cm⁻¹; MS (ES) m/e 451.1 (M+Na)⁺, 446.2 (M+NH₄)⁺, 429.2 (M+H)⁺.

### i) Ethyl (±)-10,11-dihydro-3-carboxy-5H-dibenzo[a,d]cycloheptene-10-acetate

A mixture of ethyl (±)-10,11-dihydro-3-(trifluoromethanesulfonyloxy)-5H-dibenzo[a,d]cycloheptene-10-acetate (Preparation 1(h)) (808.9 mg, 1.89 mmol), KOAc (742 mg, 7.56 mmol), Pd(OAc)₂ (21.2 mg, 0.095 mmol), 1,1'-bis(diphenylphosphino)ferrocene (210 mg, 0.38 mmol), and anhydrous DMSO (11 mL) was purged with carbon monoxide (three evacuation/CO flush cycles, followed by bubbling CO through the mixture for 5 min), then was stirred under a balloon of CO in an oil bath set at 70°C. After 3.5 h, the reaction was diluted with H₂O (11 mL), cooled to 0°C, and acidified with 1.0 N HCl (ca. 8 mL). CH₂Cl₂ extraction (3 x 30 mL), drying (Na₂SO₄), concentration, and reconcentration from toluene left a reddish-orange liquid (2-3 mL). Chromatography (silica gel, 3:2:0.1 EtOAc/toluene/AcOH; mixed fractions again with 1:1:0.1 EtOAc/toluene/AcOH) gave the title compound (581.9 mg, 95%) as a viscous, yellow oil which partially crystallized in high vacuum at 40°C: TLC R_{f} (3:2:0.1 EtOAc/toluene/AcOH) 0.60; ¹H NMR (250 MHz, CDCl₃) δ 7.95 (d, J = 1.5 Hz, 1H), 7.87 (dd, J = 7.8, 1.5 Hz, 1H), 7.00-7.35 (m, 5H), 4.40 (d, J = 15.2 Hz, 1H), 4.19 (q, J = 7.1 Hz, 2H), 3.97 (d, J = 15.2 Hz, 1H), 3.82-4.00 (m, 1H), 3.43 (dd, J = 15.3, 4.0 Hz, 1H), 3.07 (dd, J = 15.3, 9.5 Hz, 1H), 2.69 (dd, J = 15.8, 4.8 Hz, 1H), 2.53 (dd, J = 15.8, 9.5 Hz, 1H), 1.28 (t, J = 7.1 Hz, 3H); FTIR (CCl₄) 2357-3378 (broad), 1735, 1692, 1280 cm⁻¹; MS (ES) m/e 342.2 (M+NH₄)⁺, 325.2 (M +H)⁺, 307.2 (M+H-H₂O)⁺.

### Preparation 2

### Preparation of N-(t-butoxycarbonyl)-4.4'-bipiperidine

4,4'-Bipiperidine dihydrochloride (2.5 g, 10 mmol) was dissolved in water (10 mL) and treated with 5N sodium hydroxide to pH 8-9, diluted to 120 mL with ethanol and stirred at RT. The resulting mixture was treated with di-t-butyl dicarbonate (2.4 g, 11 mmol) in ethanol (80 mL) in one portion and the mixture stirred at RT, with periodic additions of 5N sodium hydroxide to maintain a pH of 8-9. After 5 h, the mixture was concentrated and the residue was dissolved in a mixture of 1:1 ether:water (100 mL) and the pH adjusted to 12 with 5N sodium hydroxide. The aqueous phase was extracted with ether and the organic phase was washed sequentially with brine, dilute citric acid, and water. The aqueous phase was adjusted to pH 12-13 with 5N sodium hydroxide and extracted with ether. The organic phase was washed with brine, dried (sodium sulfate), concentrated to a clear oil and dried *in vacuo* to give the title compound (1.7 g, 63%) as a solid. TLC R_{f} 0.4 (Kieselgel 60 F₂₅₄, 15:3:2 2-butanol:formic acid:water); MS (ES) m/e 268.3 [M+H]⁺.

### Preparation 3

### Preparation of 2-(Methylamino)methylbenzimidazole dihydrochloride

### a) 2-[(tert-Butoxycarbonyl)sarcosyl]aminoaniline

A solution of phenylenediamine (100 g, 0.924 mole) and Boc-sarcosine (175 g, 0.924 mole) in DMF (1750 mL) was cooled to -10°C under argon, and a solution of DCC (190.8 g, 0.924 mole) in CH₂Cl₂ (1750 mL) was added in a slow stream over 1 hr. The temperature rose to 0°C during the addition. The reaction was stirred overnight while the temperature was allowed to rise to RT. The white precipitate was removed by filtration, and the filtrate was diluted with H₂O (3.5 L) and saturated brine (1 L). The CH₂Cl₂ layer was separated and the aqueous phase was extracted with EtOAc (2 x 1 L). The combined organic layers were washed with H₂O (1 L) and brine (0.5 L), then were concentrated to a yellow residue (341 g). This was triturated with EtOAc to afford the title compound (179.4 g, 70%): mp 134 - 136°C.

### b) 2-[(N-tert-Butoxycarbonyl-N-methyl)aminomethyl]benzimidazole

A solution of 2-[(tert-butoxycarbonyl)sarcosyl]aminoaniline (Preparation 3(a)) (178.4 g, 0.639 mole) in THF (900 mL) and AcOH (900 mL) was heated to reflux under argon for 1 hr, then a vacuum was carefully applied to the reaction, and most of the THF was removed by distillation. The residual solution was poured into stirred ice water, and conc. NH₄OH (1150 mL) was added to adjust the pH to 10. An oil formed which crystallized on stirring overnight. The solid was filtered and dried at 50°C at atmospheric pressure for two days to leave a yellow-white solid (167 g, 100%): mp 140 - 150°C. Further drying at RT and atmospheric pressure gave the crude title compound (162 g, 97%).

### c) 2-(Methylaminomethyl)benzimidazole dihydrochloride

A solution of 4 M HCI/dioxane (616 mL, 2.46 mole) and anisole (134 mL, 1.23 mole) was cooled to 0°C under argon, and a solution of 2-[(N-tert-butoxycarbonyl-N-methyl)aminomethyl]benzimidazole (Preparation 3(b)) (161 g, 0.616 mole) in CH₂Cl₂ (800 mL) was added in a slow stream over 30 min. The temperature rose to 8°C during the addition, and a white precipitate began to form before the addition was complete. The reaction was stirred for 20 min, then the title compound (66.6 g, 46%) was collected by filtration: mp 250 - 255 °C (dec.). Anal. Calcd for C₉H₁₁N₃ · 2 HCI: C, 46.17; H, 5.60; N, 17.95. Found: C, 46.33; H, 5.68; N, 17.55. The filtrate was diluted with Et₂O, and the mixture was allowed to stand overnight. Filtration gave additional title compound (62 g; total yield 128.6 g, 89%) as a pink solid: mp 248 - 253°C (dec.).

### Preparation 4

### Preparation of 2-[(2-aminoethyl)amino]pyridine dihydrochloride

### a) Mono-Boc-1,2-ethylenediamine

A solution of di-*tert*-butyl dicarbonate (10.91 g, 50 mmole) in CH₂Cl₂ (50 mL) was added dropwise over 30 min to a briskly stirred solution of 1,2-ethylenediamine (33 mL, 500 mmole) in CH₂Cl₂ (250 mL) at 0°C under argon. A precipitate separated during the addition. When the addition was complete, the reaction was warmed to RT, stirred for 1 hr, and concentrated on the rotavap. The residue was taken up in H₂O (100 mL) and filtered to remove a small amount of insoluble material. The filtrate was extracted with CH₂Cl₂ (3 x 100 mL), and the combined organics were dried (MgSO₄) and concentrated to afford the title compound (6.00 g, 75 %) as a cloudy liquid: ¹H NMR (250, CDCl₃) δ 4.75 - 5.00 (m, 1 H), 3.05 - 3.25 (m, 2 H), 2.65 - 2.85 (m, 2 H), 1.46 (s, 9 H), 1 12 (br s, 2 H).

### b) 2-[[2-(Boc-amino)ethyl]amino]pyridine-N-oxide

A mixture of mono-Boc-1,2-ethylenediamine (Preparation 4(a)) (5.83 g, 36.39 mmole), 2-chloropyridine-N-oxide hydrochloride (7.25 g, 43.67 mmole), NaHCO₃ (15.29 g, 182 mmole), and *tert*-amyl alcohol (36 mL) was heated at reflux. After 47 hr, the dark brown mixture was cooled, diluted with CH₂Cl₂ (100 mL), and suction filtered to remove insoluble materials. The filtrate was concentrated and reconcentrated from toluene. Silica gel chromatography (10% MeOH/CHCl₃) gave impure title compound (8.23 g, 89%) as a yellow solid which was used without further purification: TLC (10% MeOH/CHCl₃) R_{f} 0.42; ¹H NMR (250, CDCl₃) δ 8.16 (dd, J = 6.5, 1.3 Hz, 1 H), 7.05 - 7.30 (m, 2 H), 6.68 (br d, J = 8.6 Hz, 1 H), 6.50 - 6.65 (m, 1 H), 5.70 - 5.95 (m, 1 H), 3.25 - 3.60 (m, 4 H), 1.44 (s, 9 H); MS (ES) m/e 254 (M + H)⁺.

### c) 2-[[2-(Boc-amino)ethyl]amino]pyridine

10% Pd/C (106.4 mg, 0.10 mmole) was added to a solution of 2-[[2-(Boc-amino)ethyl]amino]pyridine-N-oxide (Preparation 4(b)) (126.7 mg, 0.5 mmole) and cyclohexene (0.25 mL, 0.25 mmole) in absolute EtOH (5 mL), and the mixture was heated to reflux. After 16 hr, the reaction was filtered through celite® and the filtrate was concentrated. The residue was combined with the residue obtained from a separate preparation (0.5 mmole scale), and the combined materials were purified by silica gel chromatography (5% MeOH/CHCl₃). The title compound (148.4 mg, 63% based on 1 mmole of 2-[[2-(Boc-amino)ethyl]amino]pyridine-N-oxide) was obtained as a yellow oil: TLC (5% MeOH/CHCl₃) R_{f} 0.43; ¹H NMR (400, CDCl₃) δ 8.05 - 8.12 (m, 1 H), 7.37 - 7.46 (m, 1 H), 6.53 - 6.61 (m, 1 H), 6.41 (d, J = 8.3 Hz, 1 H), 5.12 (br s, 1 H), 4.86 (br s, 1 H), 3.26 - 3.51 (m, 4 H), 1.44 (s, 9 H); MS (ES) m/e 238 (M + H)⁺.

### d) 2-[(2-Aminoethyl)amino]pyridine dihydrochloride

4 N HCl in dioxane (3.2 mL) was added in a stream to a solution of 2-[[2-(Boc-amino)ethyl]amino]pyridine (Preparation 4(c)) (148.4 mg, 0.63 mmole) in anhydrous CH₂Cl₂ (3.2 mL) at 0°C, then the reaction was warmed to RT. After 2 hr, the mixture was suction filtered to collect the precipitated solid, which was washed with anhydrous Et₂O and dried to afford the title compound (132.8 mg, quantitative) as a yellow solid: ¹H NMR (400, CD₃OD) δ 7.99 - 8.07 (m, 1 H), 7.92 - 7.98 (m, 1 H), 7.19 (d, J = 9.1 Hz, 1 H), 6.98 - 7.04 (m, 1 H), 3.76 (t, J = 6.2 Hz, 2 H), 3.27 (t, J = 6.2 Hz, 2 H, partially obscured by residual solvent signal); MS (ES) m/e 138 (M + H)⁺.

### Preparation 5

### Preparation of 2-[(3-hydroxy-1-propyl)amino]pyridine-N-oxide

### a) 2-[(3-Hydroxy-1-propyl)amino]pyridine-N-oxide

A mixture of 2-chloropyridine-N-oxide (16.6 g, 0.1 mole), 3-amino-1-propanol (15.3 mL, 0.2 mole), NaHCO₃ (42 g, 0.5 mole), and *tert*-amyl alcohol (100 mL) was heated to reflux. After 21 hr, the reaction was cooled, diluted with CH₂Cl₂ (300 mL), and suction filtered to remove insoluble materials. The filtrate was concentrated and reconcentrated from toluene to leave a yellow oil. Silica gel chromatography (20% MeOH/CHCl₃) gave the title compound (15.62 g, 93%) as a yellow solid: TLC (20% MeOH/CHCl₃) R_{f} 0.48; ¹H NMR (250, CDCl₃) δ 8.07 (dd, J = 6.6, 1.2 Hz, 1 H), 7.34 (br t, 1 H), 7.10 - 7.30 (m, 1 H), 6.64 (dd, J = 8.5, 1.4 Hz, 1 H), 6.40 - 6.60 (m, 1 H), 4.49 (br s, 1 H), 3.65 - 3.90 (m, 2 H), 3.35 - 3.60 (m, 2 H), 1.75 - 2.00 (m, 2 H); MS (ES) m/e 169 (M+ H)⁺.

### Preparation 6

### Methyl 2-carboxy-6,11-dihydro-5H-dibenz[b,e]azepine-6-acetate

### a) 2-Benzyl-4-bromo-N-(formyl)aniline

Using the general procedure of *Coll. Czech. Chem. Common*. **1965**, *30*, 1163-1172, 2-benzyl-4-bromo-aniline, prepared as in *Khim. Geterotsikl. Soedin*. **1983**, *3*, 411-414, is heated with ethyl formate to afford the title compound.

### b) 2-Bromo-11H-dibenz[b,e]azepine

Using the general procedure of *Coll. Czech. Chem. Commun*. **1965**, *30*, 1163-1172, the compound of Preparation 6(a) is heated in a mixture of polyphosphoric acid and phosphorous oxychloride to afford the title compound.

### c) Methyl 2-bromo-6,11-dihydro-5H-dibenz[b,e]azepine-6-acetate

Using the general procedure of *Bull. Chem. Soc. Jpn.* **1990**, *63*, 3122-3131, the compound of Preparation 6(b) is treated with the t-butyldimethylsilyl ketene acetal of methyl acetate, trimethylsilylcyanide and a catalytic amount of di-µ-chloro-bis(1,5-cyclooctadiene)-dirhodium ([Rh(COD)Cl]₂) in dichloromethane in the cold to afford the title compound.

### d) Methyl 2-carboxy-6,11-dihydro-5H-dibenz[b,e]azepine-6-acetate

Using the general procedure of Preparation 1(i), except substituting the compound of Preparation 6(c) for the compound of Preparation 1(h), gives the title compound.

The following compounds illustrate methods for preparing the biologically active compounds of this invention from intermediate compounds such as described in the foregoing Preparations.

### Example 1

### Preparation of (±)-10,11-dihydro-3-[[[(1H-benzimidazol-2-yl)methyl]methylamino] carbonyl]-5H-dibenzo[a,d]cycloheptene-10-acetic acid

### a) Ethyl (±)-10,11-dihydro-3-[[[(1H-benzimidazol-2-yl)methyl]methylamino] carbonyl]-5H-dibenzo[a,d]cycloheptene-10-acetate

EDC (202.4 mg, 1.06 mmol) was added all at once to a solution of ethyl (±)-10,11-dihydro-3-carboxy-5H-dibenzo[a,d]cycloheptene-10-acetate (Preparation 1) (285.6 mg, 0.88 mmol), 2-(methylamino)methylbenzimidazole dihydrochloride (Preparation 3) (247.2 mg, 1.06 mmol), HOBt · H₂O (142.7 mg, 1.06 mmol), and diisopropylethylamine (0.61 mL, 3.52 mmol) in anhydrous DMF (4.4 mL) at RT. The reaction was stirred at RT for 16.5 h, then was concentrated on the rotavap (high vacuum). The residue was reconcentrated from xylenes, then was dissolved in H₂O (5 mL). EtOAc extraction (2 x 5 mL), drying (MgSO₄), concentration, and chromatography (silica gel, 5% MeOH in 1:1 EtOAc/CHCl₃) gave the title compound as a faintly yellow foam (389.2 mg, 95%): TLC R_{f} (10% MeOH in 1:1 EtOAc/CHCl₃) 0.60; ¹H NMR (400 MHz, CDCl₃) δ 7.70-7.80 (m, 1H), 7.40-7.50 (m, 1H), 7.35 (s, 1H), 7.21-7.32 (m, 3H), 7.04-7.21 (m, 5H), 4.76-4.88 (m, 2H), 4.36 (d, J = 15.2 Hz, 1H), 4.18 (q, J = 7.1 Hz, 2H), 3.90 (d, J = 15.2 Hz, 1H), 3.82-3.95 (m, 1H), 3.38 (dd, J = 15.4, 4.0 Hz, 1H), 3.11 (s, 3H), 3.03 (dd, J = 15.4, 9.5 Hz, 1H), 2.69 (dd, J = 15.8, 4.8 Hz, 1H), 2.52 (dd, J = 15.8, 9.6 Hz, 1H), 1.27 (t, J = 7.1 Hz, 3H); FTIR (CCl₄) 2700-3470 (broad), 1735, 1629 (shoulder), 1617, 1484, 1454, 1422, 1397, 1271, 1180, 1157 cm⁻¹; MS (ES) m/e 468.2 (M+H)⁺.

### b) (±)-10,11-Dihydro-3-[[[(1H-benzimidazol-2-yl)methyl]methylamino]carbonyl]-5H-dibenzo[a,d]cycloheptene-10-acetic acid, sodium salt

1.0 N LiOH (1.0 mL, 1.0 mmol) was added to a mixture of ethyl (±)-10,11-dihydro-3-[[[(1H-benzimidazol-2-yl)methyl]methylamino]carbonyl]-5H-dibenzo[a,d]cycloheptene-10-acetate (Example 1(a)) (389.2 mg, 0.83 mmol) in THF (4.2 mL) and H₂O (3.2 mL), and the resulting yellow solution was stirred at RT for 2 h, at 40°C for 15 h, then at reflux for 0.5 h. The reaction was then concentrated to dryness on the rotavap, and the residue was dissolved in H₂O (4 mL). The solution was washed with Et₂O (2 x 4 mL), and the Et₂O layers were discarded. The aqueous layer was filtered to remove particulates, then was neutralized with 1.0 N HCI (1.0 mL). The precipitated solid was collected by suction filtration and washed with H₂O, then was dissolved in hot 1:1 CH₃CN/H₂O. The solution was hot filtered to remove an insoluble brown oil, and was allowed to cool to RT. Since the product oiled out, the mixture was concentrated to dryness on the rotavap. The residue was dissolved in MeOH (2 mL), and 5% NaHCO₃ (2 mL) was added . The mixture was warmed until a homogeneous solution was produced, then was concentrated to remove the MeOH. ODS chromatography (30% MeOH/H₂O, then rechromatography with 1:1 MeOH/H₂O), concentration, and lyophilization gave the title compound as a colorless powder (187.5 mg, 45%): HPLC k' 1.47 (Hamilton PRP-1®, 35% CH₃CN/H₂O-0.1% TFA); ¹H NMR (400 MHz, CD₃OD) Rotameric mixture; 6.80-7.65 (m, 11H), 4.64-5.05 (m, 2H), 3.68-4.41 (m, 3H), 2.87-3.46 (m, 5H), 2.30-2.58 (m, 2H); MS (ES) m/e 440.2 (M +H)⁺. Anal. Calcd for C₂₇H₂₄N₃O₃Na · 2.25 H₂O: C, 64.60; H, 5.72; N, 8.37. Found: C, 64.52; H, 5.80; N, 8.27.

### Example 2

### Preparation of (±)-10,11-dihydro-3-[1-(4,4'-bipiperidinyl)carbonyl]-5H-dibenzo[a,d]cycloheptene-10-acetic acid

### a) Ethyl (±)-10,11-dihydro-3-[1-(1'-BOC-4,4'-bipiperidinyl)carbonyl]-5H-dibenzo[a,d]cycloheptene-10-acetate

EDC (209.3 mg, 1.09 mmol) was added all at once to a solution of ethyl (±)-10,11-dihydro-3-carboxy-5H-dibenzo[a,d]cycloheptene-10-acetate (Preparation 1) (296.3 mg, 0.91 mmol), 1-BOC-4,4'-bipiperidine (Preparation 2) (293 mg, 1.09 mmol), HOBt· H₂O (147.6 mg, 1.09 mmol), and diisopropylethylamine (0.32 mL, 1.82 mmol) in anhydrous DMF (4.6 mL) at RT. The reaction was stirred at RT overnight, then was concentrated on the rotavap (high vacuum). The residue was reconcentrated from xylenes, then was dissolved in H₂O (5 mL). EtOAc extraction (2 x 5 mL), drying (MgSO₄), concentration, and silica gel chromatography (7:3 EtOAc/hexanes) gave the title compound as a light yellow foam (463.4 mg, 89%): TLC R_{f} (7:3 EtOAc/hexanes) 0.59; ¹H NMR (250 MHz, CDCl₃) δ 6.94-7.46 (m, 7H), 4.58-4.88 (m, 1H), 4.37 (d, J = 15.2 Hz, 1H), 4.19 (q, J = 7.1 Hz, 2H), 3.89 (d, J = 15.2 Hz, 1H), 3.68-4.30 (m, 4H), 3.38 (dd, J = 15.3, 4.1 Hz, 1H), 3.01 (dd, J = 15.3, 9.4 Hz, 1H), 2.40-3.09 (m, 5H), 1.45 (s, 9H), 1.27 (t, J = 7.1 Hz, 3H), 0.85-1.90 (m, 11H); FTIR (CCl₄) 1734, 1694, 1637, 1426, 1171 cm⁻¹; MS (ES) m/e 1149.8 (2M+H)⁺, 597.4 (M+Na)⁺, 575.4 (M+H)⁺, 519.4 (M+H-C₄H₈)⁺.

### b) (±)-10,11-Dihydro-3-[1-(4,4'-bipiperidinyl)carbonyl]-5H-dibenzo[a,d]cycloheptene-10-acetic acid

A heterogeneous mixture of ethyl (±)-10,11-dihydro-3-[1-(1'-BOC-4,4'-bipiperidinyl)carbonyl]-5H-dibenzo[a,d]cycloheptene-10-acetate (Example 2(a)) (463.4 mg, 0.81 mmol), 1.0 N LiOH (1.0 mL, 1.0 mmol), THP (4.1 mL), and H₂O (3.1 mL) was stirred at 40°C. After 17 h, the homogeneous solution was cooled in ice and acidified with 1.0 N HCl (1.5 mL). CH₂Cl₂ extraction (3 x 10 mL), drying (MgSO₄), and concentration left an off-white foam. This was dissolved in CH₂Cl₂ (4.1 mL), and the solution was cooled to 0°C. TFA (4.1 mL) was added all at once, and the reaction was warmed to RT. After 1.5 h, the light yellow solution was concentrated to dryness on the rotavap to leave an oil. ODS chromatography (30% CH₃CN/H₂O-0.1% TFA), concentration, and lyophilization gave the title compound as a colorless powder (437.2 mg, 86%): HPLC k' 1.91 (Hamilton PRP-1®, 30% CH₃CN/H₂O-0.1% TFA); ¹H NMR (400 MHz, CD₃OD) δ 7.02-7.29 (m, 7H), 4.53-4.73 (m, 1H), 4.34 (d, J = 15.0 Hz, 1H), 3.99 (d, J = 15.0 Hz, 1H), 3.65-3.89 (m, 2H), 3.30-3.50 (m, 3H), 2.70-3.15 (m, 5H), 2.68 (dd, J = 16.0, 5.2 Hz, 1H), 2.52 (dd, J = 16.0, 9.1 Hz, 1H), 1.06-2.09 (m, 10H); MS (ES) m/e 447.2 (M +H)⁺. Anal. Calcd for C₂₈H₃₄N₂O₃ · 1.5 CF₃CO₂H · 0.75 H₂O: C, 59.00; H, 5.91; N, 4.44. Found: C, 58.96; H, 6.00; N, 4.50.

### Example 3

### Preparation of (±)-10,11-dihydro-3-[3-(2-benzimidazolyl)-1-propyl]-5H-dibenzo[a,d]cycloheptene-10-acetic acid

### a) 4-(2-Tetrahydropyranyloxy)-1-tributylstannyl-1-butyne

A solution of n-butyllithium in hexanes (1.6 M, 18.8 mL, 30 mmole) was added in a stream over 2 min to a solution of 2-(3-butynyloxy)tetrahydro-2*H*-pyran (4.7 mL, 30 mmole) in dry THF (60 mL) at 0°C under argon. After 0.5 hr, tributyltin chloride (8.1 mL, 30 mmole) was added all at once, and the reaction was warmed to RT. After 3 hr, the reaction was diluted with hexanes (300 mL) and washed sequentially with H₂O (2 x 60 mL), 10% KF (2 x 30 mL), and saturated brine (60 mL). Drying (Na₂SO₄), concentration, and silica gel chromatography (3% EtOAc/hexanes) gave the title compound (3.58 g, 27%) as a nearly colorless oil: TLC (5% EtOAc/hexanes) R_{f} 0.37; ¹H NMR (400 MHz, CDCl₃) δ 4.66 (narrow t, 1 H), 3.75 - 3.96 (m, 2 H), 3.49- 3.62 (m, 2 H), 2.56 (app t, 2 H), 1.76 - 1.91 (m, 1 H), 1.65 - 1.78 (m, 1 H), 1.42 -1.65 (m, 10 H), 1.22 - 1.41 (m, 6 H), 0.82 - 1.08 (m, 15 H).

### b) Ethyl (±)-3-[4-(2-tetrahydropyranyloxy)-1-butyn-1-yl]-5H-dibenzo[a,d]cycloheptene-10-acetate

A mixture of ethyl (±)-10,11-dihydro-3-(trifluoromethanesulfonyloxy)-5H-dibenzo[a,d]cycloheptene-10-acetate (Preparation 1(h)) (1.34 g, 3.13 mmole), 4-(2-tetrahydropyranyloxy)-1-tributylstannyl-1-butyne (Example 3(a)) (1.66 g, 3.76 mmole), LiCl (398 mg, 9.39 mmole), bis(triphenylphosphine)palladium dichloride (110 mg, 0.094 mmole), and anhydrous dioxane (31 mL) was heated at reflux under argon. After 1.5 hr, the reaction was concentrated to remove most of the dioxane, and the residue was taken up in Et₂O (100 mL). 10% KF (50 mL) was added and the mixture was stirred briskly for 0.5 hr. The aqueous layer was removed and the Et₂O layer was filtered through a mixture of celite® and MgSO₄. The filtrate was concentrated and the residue was chromatographed on silica gel (10% EtOAc/hexanes) to afford the title compound (1.12 g, 83%) as a pale yellow oil: TLC (20% EtOAc/hexanes) R_{f} 0.40; ¹H NMR (400 MHz, CDCl₃) δ 7.21 - 7.30 (m, 1 H), 7.06 -7.20 (m, 5 H), 7.00 (d, J = 7.8 Hz, 1 H), 4.69 (t, J = 3.6 Hz, 1 H), 4.31 (d, J = 15.2 Hz, 1 H), 4.11 - 4.23 (m, 2 H), 3.76 - 3.97 (m, 4 H), 3.59 - 3.68 (m, 1 H), 3.48 - 3.57 (m, 1 H), 3.34 (dd, J = 15.2, 4.1 Hz, 1 H), 2.97 (dd, J = 15.2, 9.5 Hz, 1 H), 2.70 (t, J = 7.3 Hz, 2 H), 2.65 (dd, J = 15.7, 4.8 Hz, 1 H), 2.51 (dd, J = 15.7, 9.5 Hz, 1 H), 1.78 - 1.92 (m, 1 H), 1.68 - 1.78 (m, 1 H), 1.44 - 1.68 (m, 4 H), 1.27 (t, J = 7.1 Hz, 3 H); MS (ES) m/e 455 (M + Na)⁺.

### c) Ethyl (±)-3-[4-(2-tetrahydropyranyloxy)-1-butyl]-5H-dibenzo[a,d]cycloheptene-10-acetate

A mixture of ethyl (±)-3-[4-(2-tetrahydropyranyloxy)-1-butyn-1-yl]-5H-dibenzo[a,d]cycloheptene-10-acetate (Example 3(b)) (1.2 g, 2.77 mmole), 10% Pd/C (0.3 g, 0.28 mmole), and EtOAc (28 mL) was shaken at RT under hydrogen (50 psi) on a Parr apparatus. After 3 hr, the reaction was filtered through celite® and the filtrate was concentrated. Silica gel chromatography (10% EtOAc/hexanes) gave the title compound (1.06 g, 88%) as a colorless oil: TLC (20% EtOAc/hexanes) R_{f} 0.51; ¹H NMR (400 MHz, CDCl₃) δ 7.05 - 7.20 (m, 4 H), 6.92 - 7.03 (m, 3 H), 4.53 - 4.60 (m, 1 H), 4.34 (d, J = 15.1 Hz, 1 H), 4.12 - 4.26 (m, 2 H), 3.80 - 3.90 (m, 3 H), 3.71 - 3.80 (m, 1 H), 3.44 - 3.53 (m, 1 H), 3.35 - 3.44 (m, 1 H), 3.33 (dd, J = 15.1, 4.1 Hz, 1 H), 2.95 (dd, J = 15.1, 9.4 Hz, 1 H), 2.65 (dd, J = 15.5, 4.9 Hz, 1 H), 2.49 - 2.61 (m, 3 H), 1.77 - 1.90 (m, 1 H), 1.45 - 1.77 (m, 9 H), 1.27 (t, J = 7.1 Hz, 3 H); MS (ES) m/e 459 (M + Na)⁺.

### d) Ethyl (±)-3-[4-hydroxy-1-butyl]-5H-dibenzo[a,d]cycloheptene-10-acetate

A solution of ethyl (±)-3-[4-(2-tetrahydropyranyloxy)-1-butyl]-5H-dibenzo[a,d]cycloheptene-10-acetate (Example 3(c)) (456.0 mg, 1.04 mmole) and *p*-toluenesulfonic acid monohydrate (60 mg, 0.31 mmole) in absolute EtOH (10 mL) was stirred at RT. After 2 hr, the reaction was quenched with 5 % NaHCO₃ (1 mL) and concentrated to remove the EtOH. The residue was diluted with H₂O (2 mL) and extracted with CH₂Cl₂. Drying (MgSO₄), concentration, and silica gel chromatography (1:1 EtOAc/hexanes) gave the title compound (342.4 mg, 93%) as a colorless oil: TLC (1:1 EtOAc/hexanes) R_{f} 0.49; ¹H NMR (250 MHz, CDCl₃) δ 6.85 - 7.25 (m, 7 H), 4.34 (d, J = 15.1 Hz, 1 H), 4.08 - 4.30 (m, 2 H), 3.75 - 3.95 (m, 2 H), 3.53 - 3.72 (m, 2 H), 3.33 (dd, J = 15.1, 4.1 Hz, 1 H), 2.95 (dd, J = 15.1, 9.4 Hz, 1 H), 2.40 - 2.75 (m, 4 H), 1.45 - 1.80 (m, 4H), 1.27 (t, J = 7.1 Hz, 3 H); MS (ES) m/e 353 (M + H)⁺.

### e) Ethyl (±)-3-[3-carboxy-1-propyl]-5H-dibenzo[a,d]cycloheptene-10-acetate

A solution of ethyl (±)-3-[4-hydroxy-1-butyl]-5H-dibenzo[a,d]cycloheptene-10-acetate (Example 3(d)) (342.4 mg, 0.97 mmole) in anhydrous CH₂Cl₂ (19 mL) was cooled to 0°C under argon, and 2,2,6,6-tetramethyloxopiperidinium chloride (*J. Org. Chem*. **1985**, *50*, 1332-1334; 260 mg, 1.36 mmole) was added in one portion. The reaction was stirred at 0°C for 1 hr, then 2-methyl-2-butene (1.2 mL, 11 64 mmole) was added, followed by a cold (0°C) solution of NaClO₂ (0.88 g, 7.76 mmole) and NaH₂PO₄ (0.90 g, 6.50 mmole) in H₂O (26 mL). After 10 min, the reaction was diluted with EtOAc (100 mL) and the layers were separated. The organic layer was washed sequentially with cold 1.0 N HCl (10 mL) and saturated brine (20 mL), dried (MgSO4), and concentrated. Silica gel chromatography (gradient: 1:1 EtOAc/CHCl₃ then 9:9:2 EtOAc/CHCl₃/EtOH) gave impure title compound. Rechromatography on silica gel (7:3:0.1 toluene/EtOAc/AcOH) gave the pure title compound (233.7 mg, 66%): TLC (1:1 EtOAc/CHCl₃) R_{f} 0.46; ¹H NMR (400 MHz, CDCl₃) δ 7.05 - 7.22 (m, 4 H), 6.92 - 7.05 (m, 3 H), 4.34 (d, J = 15.0 Hz, 1 H), 4.10 - 4.25 (m, 2 H), 3.80 - 3.90 (m, 2 H), 3.33 (dd, J = 15.1, 4.1 Hz, 1 H), 2.95 (dd, J = 15.1, 9.4 Hz, 1 H), 2.48 - 2.60 (m, 4 H), 2.48 - 2.60 (m, 4 H), 2.37 (t, J = 7.4 Hz, 2 H), 1.87 - 2.00 (m, 2 H), 1.27 (t, J = 7.2 Hz, 3 H); MS (ES) m/e 389 (M + Na)⁺, 367 (M + H)⁺.

### f) Ethyl (±)-3-[3-[[(2-aminophenyl)amino]carbonyl]prop-1-yl]-5H-dibenzo[a,d]cycloheptene-10-acetate

A solution of ethyl (±)-3-[3-carboxy-1-propyl]-5H-dibenzo[a,d]cycloheptene-10-acetate (Example 3(e)) (233.7 mg, 0.64 mmole) in dry THF (6.4 mL) was cooled to 0°C under argon, and 4-methylmorpholine (0.14 mL, 1.28 mmole) was added. The solution was stirred at 0°C for several min, then isobutyl chloroformate (0.11 mL, 0.83 mmole) was added dropwise. The cloudy reaction was stirred at 0°C for 0.5 hr, then a solution of 1,2-phenylenediamine (138 mg, 1.28 mmole) in dry THF (0.6 mL) was added rapidly. The reaction was warmed to RT and stirred for 3 hr, then was diluted with H₂O (2 mL) and extracted with EtOAc. Drying (MgSO₄), concentration, and silica gel chromatography (3:2 EtOAc/hexanes) gave the title compound (257.6 mg, 88%) as a light yellow foam: TLC (3:2 EtOAc/hexanes) R_{f} 0.40; ¹H NMR (250 MHz, CDCl₃) δ 6.90 - 7.23 (m, 10 H), 6.72 - 6.80 (m, 2 H), 4.33 (d, J = 15.0 Hz, 1 H), 4.10 - 4.25 (m, 2 H), 3.71 - 3.91 (m, 4 H), 3.32 (dd, J = 15.1, 4.0 Hz, 1 H), 2.95 (dd, J = 15.1, 9.5 Hz, 1 H), 2.59 - 2.72 (m, 3 H), 2.54 (dd, J = 15.6, 9.5 Hz, 1 H), 2.34 (t, J = 7.4 Hz, 2 H), 1.98 - 2.09 (m, 2 H), 1.27 (t, J = 7.1 Hz, 3 H); MS (ES) m/e 457 (M + H)⁺.

### g) Ethyl (±)-10,11-dihydro-3-[3-(2-benzimidazolyl)-1-propyl]-5H-dibenzo[a,d]cycloheptene-10-acetate

A solution of ethyl (±)-3-[3-[[(2-aminophenyl)amino]carbonyl]prop-1-yl]-5H-dibenzo[a,d]cycloheptene-10-acetate (Example 3(f)) (257.6 mg, 0.56 mmole) in glacial AcOH (2.8 mL) and dry THF (2.8 mL) was heated at reflux under argon. After 3 hr, the reaction was concentrated and the residue was reconcentrated from xylenes. The resulting residue was taken up in Et₂O (30 mL) and washed sequentially with 1.0 N NaOH (2 mL), H₂O (2 mL), and saturated brine (2 mL). Drying (MgSO₄), concentration, and silica gel chromatography (2% EtOH in 1:1 EtOAc/hexanes) gave the title compound (236.3 mg, 96%) as an off-white foam: TLC (2% EtOH in 1:1 EtOAc/hexanes) R_{f} 0.34; ¹H NMR (400 MHz, CDCl₃) δ 7.30 - 7.80 (m, 2 H), 7.03 - 7.24 (m, 6 H), 6.80 - 6.95 (m, 3 H), 4.12 - 4.28 (m, 3 H), 3.77 - 3.89 (m, 1 H), 3.74 (d, J = 15.1 Hz, 1 H), 3.28 (dd, J = 15.2, 4.0 Hz, 1 H), 2.90 (dd, J = 15.2, 9.5 Hz, 1 H), 2.84 (t, J = 7.7 Hz, 2 H), 2.65 (dd, J = 15.7, 4.9 Hz, 1 H), 2.60 (t, J = 7.5 Hz, 2 H), 2.52 (dd, J = 15.7, 9.6 Hz, 1 H), 2.03 - 2.18 (m, 2 H), 1.28 (t, J = 7.1 Hz, 3 H); MS (ES) m/e 439 (M + H)⁺.

### h) (±)-10,11-Dihydro-3-[3-(2-benzimidazolyl)-1-propyl]-5H-dibenzo[a,d]cycloheptene-10-acetic acid, sodium salt

A mixture of ethyl (±)-10,11-dihydro-3-[3-(2-benzimidazolyl)-1-propyl]-5H-dibenzo[a,d]cycloheptene-10-acetate (Example 3(g)) (236.3 mg, 0.54 mmole), 1.0 N NaOH (0.65 mL, 0.65 mmole), and absolute EtOH (4.8 mL) was warmed in an oil bath preset at 50°C. After 16 hr, the reaction was concentrated to dryness and the residue was purified by ODS chromatography (gradient: 35% MeOH/H₂O, then 40% MeOH/H₂O). Concentration followed by lyophilization gave the title compound (143 mg, 58%) as a colorless powder: HPLC (PRP-1®, 40% CH₃CN/H₂O containing 0.1% TFA) K' = 1.5; ¹H NMR (400 MHz, CD₃OD) δ 7.41 - 7.49 (m, 2 H), 6.86 - 7.27 (m, 9 H), 4.24 (d, J = 14.7 Hz, 1 H), 3.87 (d, J = 14.7 Hz, 1 H), 3.73 - 3.85 (m, 1 H), 3.23 - 3.25 (m, 1 H, partially obscured by residual solvent signal), 2.80 - 2.93 (m, 3 H), 2.62 (t, J = 7.5 Hz, 2 H), 2.56 (dd, J = 14.4, 5.1 Hz, 1 H), 2.40 (dd, J = 14.4, 9.7 Hz, 1 H), 2.05 - 2.17 (m, 2 H); MS (ES) m/e 411 (M + H)⁺. Anal. Calcd for C₂₇H₂₅N₂O₂Na · 1.5 H₂O: C, 70.57; H, 6.14; N, 6.10. Found: C, 70.65; H, 5.95; N, 5.95.

### Example 4

### Preparation of (±)-10,11-dihydro-3-[[[2-(2-pyridylamino)ethyl]amino]carbonyl]-5H-dibenzo[a,d]cycloheptene-10-acetic acid

### a) Ethyl (±)-10,11-dihydro-3-[[[2-(2-pyridylamino)ethyl]amino]carbonyl]-5H-dibenzo[a,d]cycloheptene-10-acetate

EDC (112.7 mg, 0.59 mmole) was added all at once to a solution of ethyl (±)-10,11-dihydro-3-carboxy-5H-dibenzo[a,d]cycloheptene-10-acetate (Preparation 1) (157.8 mg, 0.49 mmole), 2-[(2-aminoethyl)amino]pyridine dihydrochloride (Preparation 4) (123.5 mg, 0.59 mmole), HOBt · H₂O(79.5 mg, 0.59 mmole), and diisopropylethylamine (0.43 mL, 2.45 mmole) in anhydrous DMF (2.5 mL) at RT. The reaction was stirred at RT for 18 hr, then was concentrated. The residue was reconcentrated from xylenes, then was diluted with H₂O (5 mL) and extracted sequentially with EtOAc (2 x 5 mL) and CHCl₃ (2 x 5 mL). The organic layers were combined and treated with a little MeOH to dissolve a small amount of insoluble material. Drying (MgSO₄), concentration, and silica gel chromatography (5 % MeOH/CHCl₃) gave the title compound (199.1 mg, 92%) as a yellow oil: TLC (5% MeOH/CHCl₃) R_{f} 0.42; ¹H NMR (400, CDCl₃) δ 8.10 (d, J = 3.5 Hz, 1 H), 8.02 (br s, 1 H), 7.60 (app. narrow d, 1 H), 7.53 (app. dd, 1 H), 7.33 - 7.42 (m, 1 H), 7.08 - 7.22 (m, 5 H), 6.57 - 6.65 (m, 1 H), 6.45 (d, J = 8.3 Hz, 1 H), 4.79 - 4.90 (m, 1 H), 4.36 (d, J = 15.1 Hz, 1 H), 4.11 - 4.26 (m, 2 H), 3.92 (d, J = 15.1 Hz, 1 H), 3.80 - 3.95 (m, 1 H), 3.55 - 3.72 (m, 4 H), 3.38 (dd, J = 15.2, 4.1 Hz, 1 H), 3.03 (dd, J = 15.2, 9.5 Hz, 1 H), 2.66 (dd, J = 15.8, 4.8 Hz, 1 H), 2.50 (dd, J = 15.8, 9.6 Hz, I H), 1.27 (t, J = 7.2 Hz, 3 H); MS (ES) m/e 444 (M + H)⁺.

### b) (±)-10,11-Dihydro-3-[[[2-(2-pyridylamino)ethyl]amino]carbonyl]-5H-dibenzo[a,d]cycloheptene-10-acetic acid, sodium salt

A solution of ethyl (±)-10,11-dihydro-3-[[[2-(2-pyridylamino)ethyl]amino]carbonyl]-5H-dibenzo[a,d]cycloheptene-10-acetate (Example 4(a)) (199.1 mg, 0.45 mmole) and 1.0 N NaOH (0.54 mL, 0.54 mmole) in absolute EtOH (4 mL) was warmed in an oil bath set at 45°C. After 23 hr, the reaction was concentrated and the residue was purified by ODS chromatography (gradient: 30% MeOH/H₂O then 40% MeOH/H₂O). Concentration and lyophilization gave the title compound (95.8 mg, 46%) as a nearly colorless powder: HPLC (PRP-1®, 30% CH₃CN/H₂O containing 0.1% TFA) K'= 2.0; ¹H NMR (400 MHz, CD₃OD) δ 7.92 (app. d, J = 4.2 Hz, 1 H), 7.61 (d, J = 1.8 Hz, 1 H), 7.53 (dd, J = 7.9, 1.8 Hz, 1 H), 7.38 - 7.47 (m, 1 H), 7.01 - 7.24 (m, 5 H), 6.56 (d, J = 7.9 Hz, 1 H), 6.50 - 6.60 (m, 1 H), 4.34 (d, J = 14.9 Hz, 1 H), 3.97 (d, J = 14.9 Hz, 1 H), 3.78 - 3.89 (m, 1 H), 3.44 - 3.61 (m, 4 H), 3.39 (dd, J = 15.4, 4.4 Hz, 1 H), 3.00 (dd, J = 15.4, 10.2 Hz, 1 H), 2.61 (dd, J = 14.9, 5.1 Hz, 1 H), 2.43 (dd, J = 14.9, 9.5 Hz, 1 H); MS (ES) m/e 416 (M + H)⁺. Anal. Calcd for C₂₅H₂₄N₃O₃Na · 1.33 H₂O: C, 65.07; H, 5.82; N, 9.11. Found: C, 65.02; H, 5.62; N, 9.17.

### Example 5

### Preparation of (±)-10,11-dihydro-3-[3-(2-pyridylamino)-1-propyloxy]-5H-dibenzo[a,d]cycloheptene-10-acetic acid

### a) Ethyl (±)-10,11-dihydro-3-[3-[2-(N-oxopyridyl)amino]-1-propyloxy]-5H-dibenzo[a,d]cycloheptene- 10-acetate

A solution of 2-[(3-hydroxy-1-propyl)amino]pyridine-N-oxide (Preparation 5) (2 mmole) and diethyl azodicarboxylate (2 mmole) in anhydrous DMF (10 mL) is added slowly dropwise to a solution of ethyl (±)-10,11-dihydro-3-hydroxy-5H-dibenzo[a,d]cycloheptene-10-acetate (Preparation 1(g)) (1 mmole) and triphenylphosphine (2.1 mmole) in anhydrous DMF (10 mL) at RT under argon. When the reaction is complete, the solvents are removed on the rotavap, and the residue is reconcentrated from xylenes to remove residual DMF. Silica gel chromatography affords the title compound.

### b) Ethyl (±)-10,11-dihydro-3-[3-(2-pyridylamino)-1-propyloxy]-5H-dibenzo[a,d]cycloheptene-10-acetate

A mixture of ethyl (±)-10,11-dihydro-3-[3-[2-(N-oxopyridyl)amino]-1-propyloxy]-5H-dibenzo[a,d]cycloheptene-10-acetate (Example 5(a)) (1 mmole), cyclohexene (10 mmole), and 10% Pd/C (0.1 mmole) in isopropanol (10 mL) is heated at reflux. When the reaction is complete, the mixture is filtered through celite®, and the filtrate is concentrated on the rotavap. The residue is reconcentrated from toluene, then is chromatographed on silica gel to afford the title compound.

### c) (±)-10,11-Dihydro-3-[3-(2-pyridylamino)-1-propyloxy]-5H-dibenzo[a,d]cycloheptene -10-acetic acid, sodium salt

A mixture of ethyl (±)-10,11-dihydro-3-[3-(2-pyridylamino)-1-propyloxy]-5H-dibenzo[a,d]cycloheptene-10-acetate (Example 5(b)) (1 mmole) and 1.0 N NaOH (1.2 mmole) in absolute EtOH (10 mL) is warmed in an oil bath set at 50°C. When the reaction is complete, the solvents are removed on the rotavap and the residue is purified by ODS chromatography. Concentration and lyophilization afford the title compound.

### Example 6

### 2-[[[(1H-Benzimidazol-2-yl)methyl]methylamino]carbonyl]-6,11-dihydro-5H-dibenz[b,e]azepine-6-acetic acid

### a) Methyl 2-[[[(1H-benzimidazol-2-yl)methyl]methylamino]carbonyl]-6,11-dihydro-5H-dibenz[b,e]azepine-6-acetate

Using the procedure of Example 1(a), except substituting the compound of Preparation 6(d) for the compound of Preparation 1(i), gives the title compound.

### b) 2-[[[(1H-Benzimidazol-2-yl)methyl]methylamino]carbonyl]-6,11-dihydro-5H-dibenz[b,e]azepine-6-acetic acid

Using the procedure of Example 1(b), except substituting the compound of Example 6(a) for the compound of Example 1(a), gives the title compound.

## Claims

1. A compound according to formula (IV): 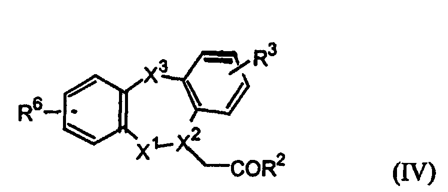 wherein
X¹-X² is CHR¹-CH, CR¹=CH, NR¹-CH, S(O)ᵤ-CH or O-CH;
X³ is CR⁵R^{5'}, NR⁵; S(O)ᵤ or O;
R' is H, C₁₋₆alkyl, C₃₋₇cycloalkyl-C₀₋₄alkyl or Ar-C₀₋₄alkyl;
R" is R', -C(O)R' or -C(O)OR⁵;
R"' is C₁₋₆alkyl, C₃₋₇cycloalkyl-C₀₋₄alkyl or Ar-C₀₋₄alkyl;
R¹ is H, C₁₋₆alkyl, C₃₋₇cycloalkyl-C₀₋₄alkyl or Ar-C₀₋₄alkyl;
R² is -OR', -NR'R", -NR'SO₂R'", -NR'OR', -OCR'₂C(O)OR', -OCR'₂OC(O)R', -OCR'₂C(O)NR'₂, CF₃ or -COCR'₂R^{2'};
R^{2'} is -OR', -CN, -S(O)ᵣR', S(O)₂NR'₂, -C(O)R'C(O)NR'₂ or -CO₂R';
R⁵ and R^{5'} are independently H, C₁₋₆alkyl, C₃₋₇cycloalkyl-C₀₋₄alkyl or Ar-C₀₋₄alkyl;
R⁶ is 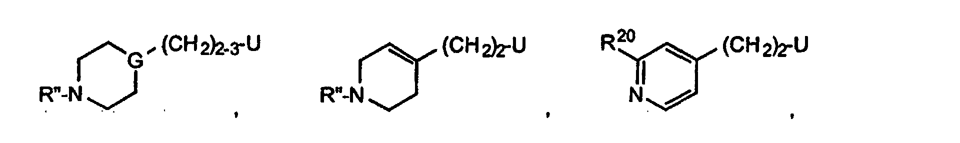 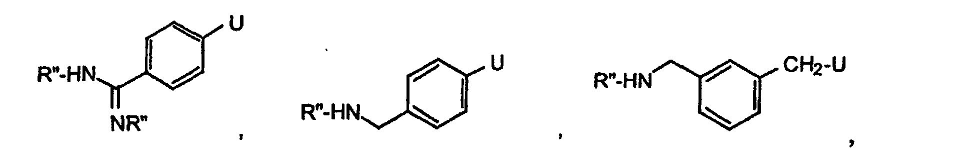 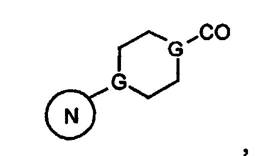
R"HNC(=NH)NH-(CH₂)₃(CHR¹⁰)-U or R"HN-(CH₂)₅-U, wherein G is N or CH, R²⁰ is hydrogen, amino, mono or di-C₁₋₄alkylamino, hydroxy or C₁₋₄alkyl and U is NR'CO, CONR', (CH₂)CO, CH=CH, C≡ C, CH₂O, OCH₂ or (CH₂)₂; or
R⁶ is W'-(CR'₂)_{q}-U- wherein W' is 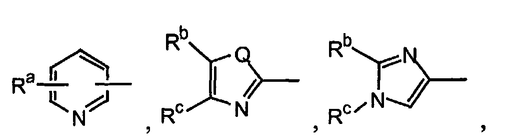 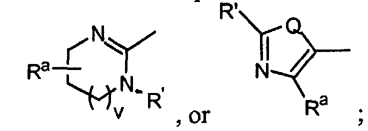
Q is NH; R^{a} is C₁₋₆alkyl, C₁₋₆alkoxy, halogen or R'NH, R^{b} and R^{c} are joined to form an optionally substituted cyclohexyl, phenyl or pyridyl ring optionally substituted by halogen, C₁₋₄alkyl, OR¹, SR¹, COR¹, OH, NO₂, N(R¹)_{2,} CO(NR¹)₂, CH₂N(R¹)₂, CN, or R"R'NC(=NR')-; and -(CR'₂)_{q}-U is (CH₂)_{q}-NR'CO, (CH₂)_{q}-CH₂O or (CH₂)_{q}-CH₂CH₂;
R³ is H, halo, -OR¹², -SR¹², -CN, -NR'R¹², -NO₂, -CF₃, CF₃S(O)ᵣ-, -CO₂R', -CONR'₂, R¹⁴-C₀₋₆alkyl-, R¹⁴-C₁₋₆oxoalkyl-, R¹⁴-C₂₋₆alkenyl-, R¹⁴-C₂₋₆alkynyl-, R¹⁴-C₀₋₆alkyloxy-, R¹⁴-C₀₋₆alkylamino- or R¹⁴-C₀₋₆alkyl-S(O)ᵣ-;
R¹⁰ is H, C₁₋₄alkyl or -NR'R";
R¹² is R', -C(O)R', -C(O)NR'₂, -C(O)OR⁵, -S(O)ₘR' or S(O)₂NR'₂;
R¹⁴ is H, C₃₋₆cycloalkyl, Het or Ar;
m is 1 or 2;
q is 0, 1, 2 or 3;
r is 0, 1 or 2;
u is 0, 1 or 2; and
v is 0 or 1;
Ar represents phenyl or naphthyl optionally substituted by one to three of C₁₋₄alkyl, C₁₋₄alkoxy, C₁₋₄alkthio, trifluoroalkyl, OH, F, Cl, Br or I;
Het represents an optionally substituted five or six membered monocyclic ring, or a nine or ten-membered bicyclic ring containing one to three heteroatoms chosen from the group of nitrogen, oxygen and sulfur optionally substituted by one to three of C₁₋₄alkyl, C₁₋₄alkoxy, C₁₋₄alkthio, trifluoroalkyl, OH, F, Cl, Br or I;
Ⓝ represents a nitrogen heterocycle, which may be a saturated or unsaturated stable five-, six- or seven-membered monocyclic ring, or a seven- to ten-membered bicyclic ring containing up to three nitrogen atoms or containing one nitrogen atom and a heteroatom chosen from oxygen and sulfur, and which may be substituted by H, C₁₋₄alkoxy, F, Cl, Br, I, NO₂, NR'₂, OH, CO₂R', CONHR', CF₃, R¹⁴-C₀₋₄alkyl, R¹⁴-C₁₋₄alkyl-S(O)ᵤ or C₁₋₄alkyl substituted by any of the aforementioned substituents;
C₃₋₇cycloalkyl refers to a carbocyclic system of three to seven carbon atoms, which may contain up to two unsaturated carbon-carbon bonds optionally substituted by up to three substituents, such as chosen from R³, on the cycloalkyl ring; or
a pharmaceutically acceptable salt thereof.

2. A compound according to claim 1, wherein X₁-X₂ is CH₂-CH or NH-CH and X₃ is CH₂.

3. A compound according to claim 1 which is:
(±)-10,11-Dihydro-3-[[[(1H-benzimidazole-2-yl)methyl]methylamino]carbonyl]-5H-dibenzo[a,d]cycloheptene-10-acetic acid;
(±)-10,11-Dihydro-3-[1-(4,4'-bipiperidinyl)carbonyl]-5H-dibenzo[a,d]cycloheptene-10-acetic acid;
(±)-10,11-Dihydro-3-[3-(2-benzimidazolyl)-1-propyl]-5H-dibenzo[a,d]cycloheptene-10-acetic acid;
(±)-10,11-Dihydro-3-[[[2-(2-pyridylamino)ethyl]amino]carbonyl]-5H-dibenzo[a,d]cycloheptene-10-acetic acid;
(±)-10,11-Dihydro-3-[3-(2-pyridylamino)-1-propyloxy]-5H-dibenzo[a,d]cycloheptene-10-acetic acid; or
2-[[[(1H-Benzimidazol-2-yl)methyl]methylamino]carbonyl]-6,11-dihydro-5H-dibenz[b,e]azepine-6-acetic acid..

4. A pharmaceutical composition comprising a compound according to claim I and a pharmaceutically acceptable carrier.

5. Use of a compound according to claim 1 in the manufacture of a medicament for inhibiting the fibrinogen receptor.

6. Use of a compound according to claim 1 in the manufacture of a medicament for inhibiting a vitronectin receptor.

7. Use of a compound according to claim I and a pharmaceutically acceptable carrier in the manufacture of a medicament for the treatment of osteoporosis, atherosclerosis, cancer or restenosis following angioplasty in a mammal.

8. Use of a compound according to claim 1 and a pharmaceutically acceptable carrier in the manufacture of a medicament for the treatment of stroke, transient ischemia attacks, myocardial infarction or inhibiting reocclusion following thrombolytic therapy.

9. A process for preparing a compound of formula (IV) as defined in claim 1, which process comprises reacting a compound of formula (X): 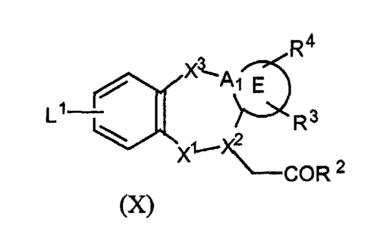 wherein X¹, X², X³, R² and R³ are as defined in formula (IV), R⁴ represents hydrogen, A₁ represents C, E represents phenyl, and L¹ is meta to the ring junction and is CHO, CO₂R', Br, I, OH, CF₃SO₃, CH₂-T or NR'R¹⁵ and T is OH, NHR¹⁵, Cl, Br or I;
with a compound of formula (XI):
R^{6'}-L² (XI)
wherein R^{6'} is W'-(CR'₂)_{q}-L², where W', R' and q are as defined in formula (IV), L² is OH, NHR¹⁵, C≡C , CHO, CO₂R', Br, I or Cl and R¹⁵ is H, C₁₋₁₀alkyl, C₃₋₇ cycloalkyl-C₀₋₈alkyl or Ar-C₀₋₈alkyl;
and thereafter removing any protecting groups, and optionally forming a pharmaceutically acceptable salt.

## Patentansprüche

1. Verbindung gemäß Formel (IV): 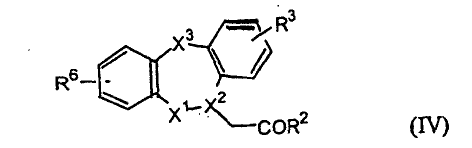 wobei
X¹-X² CHR¹-CH, CR¹=CH, NR¹-CH, S(O)ᵤ-CH oder O-CH ist;
X³ CR⁵R^{5'}, NR⁵, S(O)ᵤ oder O ist;
R' H, ein C₁₋₆-Alkyl-, C₃₋₇-Cycloalkyl-C₀₋₄-alkyl- oder Ar-C₀₋₄-Alkylrest ist;
R" R', -C(O)R' oder -C(O)OR⁵ ist;
R"' ein C₁₋₆-Alkyl-, C₃₋₇-Cycloalkyl-C₀₋₄-alkyl- oder Ar-C₀₋₄-Alkylrest ist;
R¹ H, ein C₁₋₆-Alkyl-, C₃₋₇-Cycloalkyl-C₀₋₄-alkyl- oder Ar-C₀₋₄-Alkylrest ist;
R² -OR', -NR'R", -NR'SO₂R'", -NR'OR', -OCR'₂C(O)OR', -OCR'₂OC(O)-R', -OCR'₂C(O)NR'₂, -CF₃ oder -COCR'₂R²' ist;
R^{2'} -OR', -CN, -S(O)ᵣR', -S(O)₂NR'₂, -C(O)R', -C(O)NR'₂ oder -CO₂R' ist;
R⁵ und R^{5'} unabhängig voneinander H, ein C₁₋₆-Alkyl-, C₃₋₇-Cycloalkyl-C₀₋₄-alkyl- oder Ar-C₀₋₄-Alkylrest sind;
R⁶ 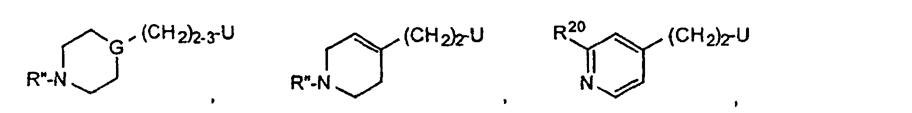 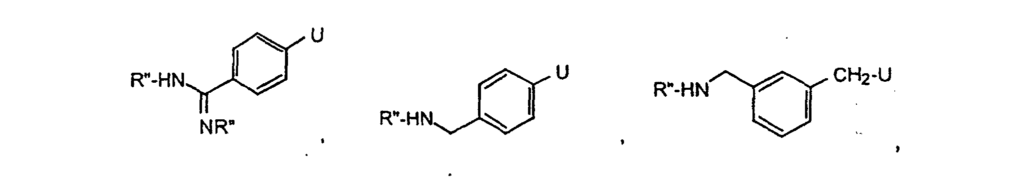 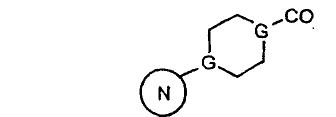
R"HNC(=NH)NH-(CH₂)₃(CHR¹⁰)-U oder R"HN-(CH₂)₅-U ist, wobei G N oder CH ist, R²⁰ ein Wasserstoffatom, eine Amino-, mono oder Di-C₁₋₄-alkylamino-, Hydroxygruppe oder ein C₁₋₄-Alkylrest ist und U NR'CO, CONR', (CH₂)CO, CH=CH, C≡C, CH₂O, OCH₂ oder (CH₂)₂ ist; oder
R⁶ W'-(CR'₂)_{q}-U- ist, wobei W' 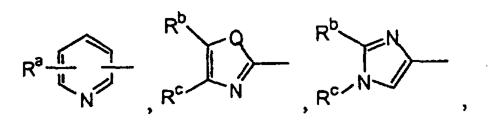 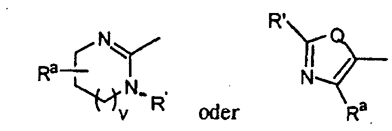 ist; Q NH ist; R^{a} ein C₁₋₆-Alkyl-, C₁₋₆-Alkoxyrest, ein Halogenatom oder R'NH ist, R^{b} und R^{c} verbunden sind, um einen gegebenenfalls substituierten Cyclohexyl-, Phenyl- oder Pyridylring zu bilden, die gegebenenfalls mit einem Halogenatom, einem C₁₋₄-Alkylrest, OR¹, SR¹, COR¹, OH, NO₂, N(R¹)₂, CO(NR¹)₂, CH₂N(R¹)₂, CN oder R"R'NC(=NR')- substituiert sind; und -(CR'₂)_{q}-U (CH₂)_{q}-NR'CO, (CH₂)_{q}-CH₂O oder (CH₂)_{q}-CH₂CH₂ ist;
R³ H, ein Halogenatom, -OR¹², -SR¹², -CN, -NR'R¹², -NO₂, -CF₃, CF₃S(O)ᵣ-, -CO₂R', -CONR'₂, ein Rest R¹⁴-C₀₋₆-alkyl-, R¹⁴-C₁₋₆-oxoalkyl-, R¹⁴-C₂₋₆-alkenyl-, R¹⁴-C₂₋₆-alkynyl-, R¹⁴-C₀₋₆-alkyloxy-, R¹⁴-C₀₋₆-alkylamino- oder R¹⁴-C₀₋₆-alkyl-S(O)ᵣ- ist;
R¹⁰ H, ein C1-4-Alkylrest oder -NR'R" ist;
R¹² R', -C(O)R', -C(O)NR'₂, -C(O)OR⁵, -S(O)ₘR' oder S(O)₂NR'₂ ist;
R¹⁴ H, ein C₃₋₆-Cycloalkylrest, Het oder Ar ist;
m 1 oder 2 ist;
q 0, 1, 2 oder 3 ist;
r 0, 1 oder 2 ist;
u 0, 1 oder 2 ist; und
v 0 oder 1 ist;
Ar eine Phenyl- oder Naphthylgruppe bedeutet, die gegebenenfalls mit einem bis drei Resten aus einem C₁₋₄-Alkyl-, C₁₋₄-Alkoxy-, C₁₋₄-Alkylthio-, Trifluoralkylrest, OH, F, Cl, Br oder I substituiert ist;
Het einen gegebenenfalls substituierten fünf- oder sechsgliedrigen monocyclischen Ring bedeutet oder einen neun- oder zehngliedrigen bicyclischen Ring bedeutet, der ein bis drei Heteroatome, ausgewählt aus einem Stickstoff-, Sauerstoff- und Schwefelatom, enthält, der gegebenenfalls mit einem bis drei Resten aus einem C₁₋₄-Alkyl-, C₁₋₄-Alkoxy-, C₁₋₄-Alkylthio-, Trifluoralkylrest, OH, F, Cl, Br oder I substituiert ist;
Ⓝ ein Stickstoffheterozyklus bedeutet, welcher ein gesättigter oder ungesättigter stabiler fünf-, sechs oder siebengliedriger monocyclischer Ring oder ein sieben- bis zehngliedriger bicyclischer Ring sein kann, der bis zu drei Stickstoffatome oder ein Stickstoffatom und ein Heteroatom, ausgewählt aus einem Sauerstoff- und Schwefelatom, enthält, und welcher mit H, einem C₁₋₄-Alkoxyrest, F, Cl, Br, I, NO₂, NR'₂, OH, CO₂R', CONHR', CF₃, einem Rest R¹⁴-C₀₋₄-alkyl-, R¹⁴-C₁₋₄-alkyl-S(O)ᵤ - oder einem C₁₋₄-Alkylrest, der mit einem der vorstehend erwähnten Substituenten substituiert ist, substituiert sein kann;
C₃₋₇-Cycloalkylrest sich auf ein carbocyclisches System mit drei bis sieben Kohlenstoffatomen bezieht, welches bis zu zwei ungesättigte Kohlenstoff-Kohlenstoff-Bindungen enthalten kann, das gegebenenfalls mit bis zu drei Substituenten, welche aus R³ ausgewählt sind, am Cycloalkylring substituiert ist; oder Ein pharmazeutisch verträgliches Salz davon.

2. Verbindung gemäß Anspruch 1, wobei X₁-X₂ CH₂-CH oder NH-CH ist und X₃ CH₂ ist.

3. Verbindung gemäß Anspruch 1, welche ist:
(±)-10,11-Dihydro-3-[[[(1H-benzimidazol-2-yl)methyl]methylamino]carbonyl]-5Hdibenzo[a,d]cyclohepten-10-essigsäure;
(±)-10,11-Dihydro-3-[1-(4,4'-bipiperidinyl)carbonyl]-5H-dibenzo[a,d]cyclohepten-10-essigsäure;
(±)-10,11-Dihydro-3-[3-(2-benzimidazolyl)-1-propyl]-5H-dibenzo[a,d]cyclohepten-10-essigsäure;
(±)-10,11-Dihydro-3-[[[2-(2-pyridylamino)ethyl]amino]carbonyl]-5Hdibenzo[a,d]cyclohepten-10-essigsäure;
(±)-10,11-Dihydro-3-[3-(2-pyridylamino)-1-propyloxy]-5H-dibenzo[a,d]cyclohepten-10-essigsäure; oder
2-[[[(1H-Benzimidazol-2-yl)methyl]methylamino]carbonyl]-6,11-dihydro-5Hdibenz[b,e]azepin-6-essigsäure.

4. Arzneimittel umfassend eine Verbindung gemäß Anspruch 1 und einen pharmazeutisch verträglichen Träger.

5. Verwendung einer Verbindung gemäß Anspruch 1 zur Herstellung eines Medikaments zur Inhibierung eines Fibrinogenrezeptors.

6. Verwendung einer Verbindung gemäß Anspruch 1 zur Herstellung eines Medikaments zur Inhibierung eines Vitronectinrezeptors.

7. Verwendung einer Verbindung gemäß Anspruch 1 und eines pharmazeutisch verträglichen Trägers zur Herstellung eines Medikaments zur Behandlung von Osteoporose, Atherosklerose, Krebs oder Restenosis in Folge einer Gefäßchirurgie bei einem Säugetier.

8. Verwendung einer Verbindung gemäß Anspruch 1 und eines pharmazeutisch verträglichen Trägers zur Herstellung eines Medikaments zur Behandlung von Schlaganfall, vorübergehenden Ischämieanfällen, Myocardinfarkt oder zur Hemmung einer Reokklusion nach einer Thrombus zersetzenden Behandlung.

9. Verfahren zur Herstellung einer Verbindung der Formel (IV), welche die in Anspruch 1 angegebene Bedeutung hat, wobei das Verfahren das Umsetzen einer Verbindung der Formel (X): 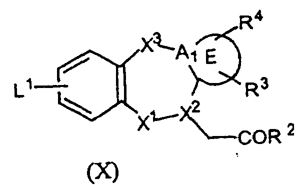 wobei X¹, X², X³, R² und R³ die in Formel (IV) angegebene Bedeutung haben, R⁴ ein Wasserstoffatom bedeutet, A₁ C bedeutet, E eine Phenylgruppe bedeutet und L¹ in meta-Position zu der Ringverbindungsstelle ist und CHO, CO₂R', Br, I, OH, CF₃SO₃, CH₂-T oder NR'R¹⁵ ist und T OH, NHR¹⁵, Cl, Br oder I ist;
mit einer Verbindung der Formel (XI):
R^{6'}-L² (XI)
wobei R^{6'} W'-(CR'₂)_{q}-L² ist, wobei W', R' und q die in Formel (IV) angegebene Bedeutung haben, L² OH, NHR¹⁵, C≡C, CHO, CO₂R', Br, I oder Cl ist und R¹⁵ H, ein C₁₋₁₀-Alkyl-, C₃₋₇-Cycloalkyl-C₀₋₈-alkyl- oder Aryl-C₀₋₈-alkylrest ist;
und nachfolgendes Entfernen der Schutzgruppen und gegebenenfalls Erzeugen eines pharmazeutisch verträglichen Salzes umfasst.

## Revendications

1. Composé de formule (IV) : 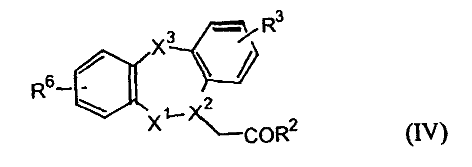 dans laquelle
X¹-X² représente un groupe CHR¹-CH, CR¹=CH, NR¹-CH, S(O)ᵤ-CH ou O-CH ;
X³ représente un groupe CR⁵R^{5'}, NR⁵, S(O)ᵤ ou O ;
R' représente H, un groupe alkyle en C₁ à C₆, (cycloalkyle en C₃ à C₇) (alkyle en C₀ à C₄) ou Ar-(alkyle en C₀ à C₄) ;
R" représente un groupe R', -C(O)R' ou -C(O)OR⁵ ;
R''' représente un groupe alkyle en C₁ à C₆, (cycloalkyle en C₃ à C₇) (alkyle en C₀ à C₄) ou Ar (alkyle en C₀ à C₄) ;
R¹ représente H, un groupe alkyle en C₁ à C₆, (cycloalkyle en C₃ à C₇) (alkyle en C₀ à C₄) ou Ar-(alkyle en C₀ à C₄) ;
R² représente un groupe -OR', -NR'R", -NR'SO₂R''', -NR'OR', -OCR'₂C(O)OR', -OCR'₂OC(O)-R', -OCR'₂C(O)NR'₂, CF₃ ou -COCR'₂R^{2'} ;
R^{2'} représente un groupe -OR', -CN, -S(O)ᵣR', S(O)₂NR'₂, -C(O)R'C(O)NR'₂ ou -CO₂R' ;
R⁵ et R^{5'} représentent, indépendamment, H, un groupe alkyle en C₁ à C₆, (cycloalkyle en C₃ à C₇) (alkyle en C₀ à C₄) ou Ar-(alkyle en C₀ à C₄) ;
R⁶ représente un groupe 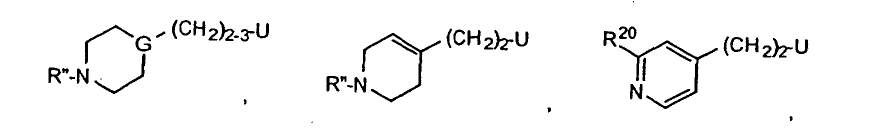 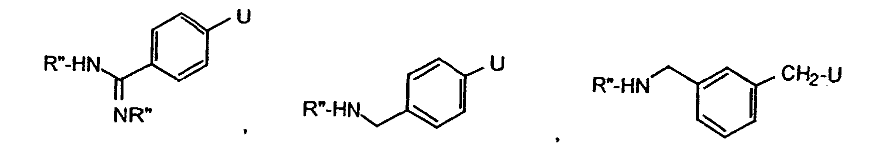 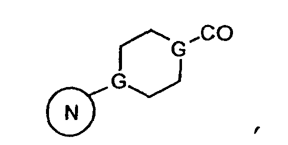
R"HNC(=NH)NH-(CH₂)₃(CHR¹⁰)-U ou R"HN-(CH₂)₅-U,
dans lequel G représente N ou un groupe CH, R²⁰ représente un atome d'hydrogène, un groupe amino, mono- ou di-(alkyle en C₁ à C₄)amino, hydroxy ou alkyle en C₁ à C₄ et U représente un groupe NR'CO, CONR', (CH₂)CO, CH=CH, C≡C, CH₂O, OCH₂ ou (CH₂)₂ ; ou
R⁶ représente un groupe W'-(CR'₂)_{q}-U- dans lequel W' représente un groupe 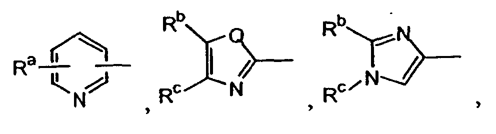 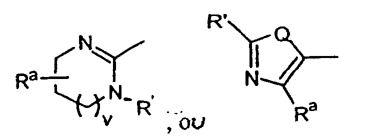 Q représente un groupe NH ; R^{a} représente un groupe alkyle en C₁ à C₆, alkoxy en C₁ à C₆, halogéno ou R'NH, R^{b} et R^{c} sont joints pour former un noyau cyclohexyle, phényle ou pyridyle facultativement substitué avec des substituants halogéno, alkyle en C₁ à C₄, OR¹, SR¹, COR¹, OH, NO₂, N(R¹)₂, CO(NR¹)₂, CH₂N(R¹)₂, CN ou R"R'NC (=NR')- ; et -(CR'₂)_{q}-U est (CH₂)_{q}-NR'CO, (CH₂)_{q}-CH₂O ou (CH₂)_{q}-CH₂CH₂ ;
R³ représente H, un groupe halogéno, -OR¹², -SR¹², -CN, -NR'R¹², -NO₂, -CF₃, CF₃S(O)ᵣ-, -CO₂R', -CONR'₂, R¹⁴-(alkyle en C₀ à C₆)-, R¹⁴-(oxoalkyle en C₁ à C₆)-, R¹⁴-(alcényle en C₂ à C₆)-, R¹⁴-(alcynyle en C₂ à C₆)-, R¹⁴-(alkyle en C₀ à C₆)oxy-, R¹⁴-(alkyle en C₀ à C₆)amino- ou R¹⁴-(alkyle en C₀ à C₆)-S(O)ᵣ- ;
R¹⁰ représente H, un groupe alkyle en C₁ à C₄ ou -NR'R" ;
R¹² représente un groupe R', -C(O)R', -C(O)NR'₂, -C(O)OR⁵, -S(O)ₘR' ou S(O)₂NR'₂ ;
R¹⁴ représente H, un groupe cycloalkyle en C₃ à C₆, Het ou Ar ;
m est égal à 1 ou 2 ;
q est égal à 0, 1, 2 ou 3 ;
r est égal à 0, 1 ou 2 ;
u est égal à 0, 1 ou 2 ; et
v est égal à 0 ou 1 ;
Ar représente un groupe phényle ou naphtyle facultativement substitué avec un à trois substituants alkyle en C₁ à C₄, alkoxy en C₁ à C₄, alkylthio en C₁ à C₄, trifluoralkyle, OH, F, Cl, Br ou I ;
Het représente un noyau monocyclique penta- ou hexagonal facultativement substitué, ou un noyau bicyclique nona- ou décagonal contenant un à trois hétéroatomes choisis dans le groupe consistant en des atomes d'azote, d'oxygène et de soufre facultativement substitué avec un à trois substituants alkyle en C₁ à C₄, alkoxy en C₁ à C₄, alkylthio en C₁ à C₄, trifluoralkyle, OH, F, Cl, Br ou I ;
Ⓝ représente un hétérocycle azoté, qui peut être un noyau monocyclique penta-, hexa- ou heptagonal saturé ou insaturé stable, ou un noyau bicyclique hepta- à décagonal contenant jusqu'à trois atomes d'azote ou contenant un atome d'azote et un hétéroatome choisi entre des atomes d'oxygène et de soufre, et qui peut être substitué avec des substituants H, alkoxy en C₁ à C₄, F, Cl, Br, I, NO₂, NR'₂, OH, CO₂R', CONHR', CF₃, R¹⁴-(alkyle en C₀ à C₄), R¹⁴-(alkyle en C₁ à C₄)-S(O)ᵤ ou alkyle en C₁ à C₄ substitué avec n'importe lequel des substituants précités ;
l'expression "cycloalkyle en C₃ à C₇) désigne un système carbocyclique de trois à sept atomes de carbone, qui peut contenir jusqu'à deux doubles liaisons carbone-carbone insaturées, facultativement substitué avec jusqu'à trois substituants, tels que ceux choisis parmi R³, sur le noyau cycloalkyle ; ou
un de ses sels pharmaceutiquement acceptables.

2. Composé suivant la revendication 1, dans lequel X₁-X₂ représente un groupe CH₂-CH ou NH-CH et X₃ représente un groupe CH₂.

3. Composé suivant la revendication 1, qui consiste en :
acide (±)-10,11-dihydro-3-[[[(1H-benzimidazole-2-yl)-méthyl]méthylamino]carbonyl]-5H-dibenzo[a,d]cycloheptène-10-acétique ;
acide (±)-10,11-dihydro-3-[1-(4,4'-bipipéridinyl)-carbonyl]-5H-dibenzo[a,d]cycloheptène-10-acétique;
acide (±)-10,11-dihydro-3-[3-(2-benzimidazolyl)-1-propyl]-5H-dibenzo[a,d]cycloheptène-10-acétique ;
acide (±)-10,11-dihydro-3-[[[2-((2-pyridylamino)-éthyl]carbonyl]-5H-dibenzo[a,d]cycloheptène-10-acétique;
acide (±)-10,11-dihydro-3-[3-(2-pyridylamino)-1-propyloxy]-5H-dibenzo[a,d]cycloheptène-10-acétique; ou
2-[[[(1H-benzimidazole-2-yl)méthyl]méthylamino]-carbonyl]-6,11-dihydro-5H-dibenz[b,e]azépine-6-acétique.

4. Composition pharmaceutique comprenant un composé suivant la revendication 1 et un support pharmaceutiquement acceptable.

5. Utilisation d'un composé suivant la revendication 1 dans la production d'un médicament destiné à l'inhibition du récepteur de fibrinogène.

6. Utilisation d'un composé suivant la revendication 1 dans la production d'un médicament destiné à inhiber un récepteur de vitronectine.

7. Utilisation d'un composé suivant la revendication 1 et d'un support pharmaceutiquement acceptable dans la production d'un médicament destiné au traitement de l'ostéoporose, de l'athérosclérose, du cancer ou d'une resténose après angioplastie chez un mammifère.

8. Utilisation d'un composé suivant la revendication 1 et d'un support pharmaceutiquement acceptable dans la production d'un médicament destiné au traitement d'un ictus, d'attaques ischémiques transitoires ou d'un infarctus du myocarde ou pour l'inhibition d'une réocclusion après une thérapeutique thrombolytique.

9. Procédé pour la préparation d'un composé de formule (IV) répondant à la définition suivant la revendication 1, procédé qui comprend la réaction d'un composé de formule (X) : 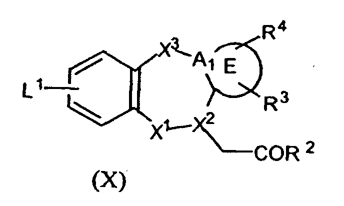 dans laquelle X¹, X², X³, R² et R³ répondent aux définitions mentionnées pour la formue (IV), R⁴ représente un atome d'hydrogène, A₁ représente C, E représente un groupe phényle et L¹ est en position méta par rapport à la jonction du noyau et représente un groupe CHO, CO₂R', Br, I, OH, CF₃SO₃, CH₂-T ou NR'R¹⁵ et T représente un groupe OH, NHR¹⁵, Cl, Br ou I ;
avec un composé de formule (XI) :
R^{6'}L² (XI)
dans laquelle R^{6'} représente un groupe W'-(CR'₂)_{q}-L², dans lequel W', R' et q répondent aux définitions mentionnées pour la formule (IV), L² représente un groupe OH, NHR¹⁵, C≡C, CHO, CO₂R', Br, I ou Ci et R¹⁵ représente H, un groupe alkyle en C₁ à C₁₀, (cycloalkyle en C₃ à C₇) (alkyle en C₀ à C₈) ou Ar-(alkyle en C₀ à C₈) ;
et ensuite l'élimination de n'importe quels groupes protecteurs et, facultativement, la formation d'un sel pharmaceutiquement acceptable.
